# EUROPEAN PATENT APPLICATION

(11) **EP 4 850 684 A1**
(43) Date of publication of application: **09.12.2026**
(21) Application number: 25748039.2
(22) Date of filing: 03.02.2025
(51) Int. Cl.: A61K 39/00, A61K 39/395, A61K 47/68, A61P 35/00, C07K 16/18, C07K 16/28, C07K 16/30, C07K 16/32

(54) **MULTISPECIFIC ANTIBODY CONTAINING LRRC15 ANTIGEN-BINDING DOMAIN**

(30) Priority: 04.02.2024 CN 202410158274
(71) Applicant: Suzhou Zelgen Biopharmaceuticals Co., Ltd., Suzhou, Jiangsu 215300 (CN); Shanghai Zelgen Pharma.Tech Co., Ltd., Shanghai 201203 (CN)
(72) Inventor: LIN, Feng, Suzhou, Jiangsu 215300 (CN); SHENG, Jackie Z, Suzhou, Jiangsu 215300 (CN); DAI, Tongcheng, Shanghai 201203 (CN); LV, Binhua, Shanghai 201203 (CN); YAN, Shude, Suzhou, Jiangsu 215300 (CN); LIBAN, Tyler, Suzhou, Jiangsu 215300 (CN); ZHANG, Bin, Suzhou, Jiangsu 215300 (CN); SHENG, Zelin, Suzhou, Jiangsu 215300 (CN)
(74) Representative: dompatent
(86) International application number: PCT/CN2025/075637
(87) International publication number: WO 2025/162477

(57) **Abstract**

The present invention relates to a multispecific antibody containing an LRRC15 antigen-binding domain and the use thereof. Particularly, provided in the present invention is a multispecific antibody, which contains a first targeting domain that targets to a leucine-rich repeat containing 15 (LRRC15) molecule highly expressed on the surface of a tumor cell, also contains a second targeting domain that binds to a CD40 molecule on the surface of an antigen presenting cell (APC) or a CD3 molecule on the surface of a T cell, and optionally, contains a third targeting domain that targets to PD-1, CLDN18.2 or FAP, and/or a fourth targeting domain that targets to TGF-β. The LRRC15-related multispecific antibody of the present invention can effectively activate T cells and reduce tumor stroma impact, thereby providing effective treatment for tumors and fibrotic diseases.

## Description

### TECHNICAL FIELD

The present invention relates to the technical field of cancer therapy, in particular, to a multispecific antibody comprising an anti-LRCC15 antibody or an immunoreactive fragment thereof for cancer treatment.

### BACKGROUND

Cancer is generally defined as a group of diseases involving abnormal cell growth with the potential to invade or spread to other parts of the body. Conventional cancer treatments are aimed at eliminating cancer tissue and preventing its spread. Such treatment options include surgery, chemotherapy, radiotherapy, hormone therapy, targeted therapy, and palliative care. Treatment is usually based on the type, location, and stage of the cancer, as well as the patient's health and preferences. However, these therapies have limitations, as they may be ineffective, especially when the cancer has already metastasized. In addition, chemotherapy and radiotherapy have a range of side effects associated with their cytotoxicity.

Currently, promising areas of cancer treatment include the use of antibodies for targeted therapy. Another promising area is the development of therapies that utilize the immune system to attack and kill tumor cells.

Leucine-rich repeat containing 15 (LRRC15) is a member of the leucine-rich repeat (LRR) superfamily. LRRC15 is highly expressed on tumor cells, including breast cancer, brain tumors, pancreatic cancer, lung cancer, melanoma, highly invasive sarcomas, and breast cancer bone metastasis, while its expression level in normal tissues is relatively low. In addition, it is also highly expressed on stromal fibroblasts in many solid tumors (e.g., breast, head and neck, lung, pancreas). Therefore, LRRC15 is a candidate for targeted therapies such as antibody drug conjugates, T cell engagers, and CAR-T cells.

CD40 and CD40L are co-stimulatory molecules linking innate immunity and adaptive immunity. Binding of CD40 on antigen-presenting cells (APCs) to CD40 ligand (CD40L), which is predominantly expressed by activated T cells, induces APC activation. In turn, APCs trigger cytotoxic T-lymphocyte (CTL) responses. CD40 antibodies can bind to and activate the CD40 signaling pathway with higher affinity, thereby activating the immune system.

Claudins are a family of four-transmembrane integral membrane proteins present on epithelial and endothelial cells. Claudin 18.2 is an isoform of Claudin 18. It is scarcely expressed in normal tissues, yet aberrantly expressed during the development and progression of a variety of primary malignant tumors, including gastric/gastroesophageal junction (GC/GEJ) cancer, breast cancer, colon cancer, head-and-neck cancer, bronchial cancer, and non-small-cell lung cancer. At present, Astellas's anti-Claudin 18.2 monoclonal antibody has completed Phase III clinical trials with promising efficacy, and is expected to become the first marketed drug against this target.

PD-1/PD-L1 represent important targets for immuno-oncology (IO) therapy. PD-L1 is highly expressed in most tumors. The binding of PD-L1 to PD-1 on the surface of T-cells delivers inhibitory signals to T-cells. Accordingly, blockade of the PD-1/PD-L1 axis can effectively activate T-cell-mediated killing of tumor cells. Since the launch of Nivolumab, the first-in-class PD-1 antibody in 2014, multiple PD-1/PD-L1 monoclonal antibodies have achieved favorable clinical therapeutic outcomes.

Fibroblast activation protein α (FAP) is a type II transmembrane serine protease. It is expressed at low levels in most tissues but exhibits elevated expression in tumors such as pancreatic cancer and breast cancer. Similar to LRRC15, FAP is predominantly expressed on cancer-associated fibroblasts (CAFs) within the stroma of most epithelial carcinomas. FAP modulates tumor growth via multiple mechanisms, including promoting tumor proliferation, migration, and angiogenesis. Accordingly, therapeutic agents targeting FAP can effectively act on tumors and their fibrotic cells to achieve therapeutic effects.

Transforming growth factor-β (TGF-β) is a multifunctional cytokine belonging to the transforming growth factor-β superfamily. It plays critical roles in regulating inflammatory processes, as well as in stem-cell differentiation and T-cell modulation and differentiation. Dysregulated TGF-β signaling disrupts immune homeostasis and promotes inflammation, fibrosis, cancer, and autoimmune diseases. TGF-β receptor II is capable of binding and sequestering TGF-β molecules, blocking TGF-β signaling and thereby inhibiting its suppressive effect on immune cells within the tumor microenvironment. Multispecific antibodies combining TGF-β receptor II with antibodies against LRRC15, FAP or the like hold promise for treating diseases such as tumors and tumor-associated fibrosis.

Despite the increasing number of therapeutic monoclonal antibodies approved for the treatment of various cancers, resistance to such antibodies is frequently observed given the multitude of distinct molecular pathways driving cancer growth and progression toward metastasis. Although the immune system constitutes a primary mechanism for cancer prevention, cancer cells evade immune surveillance. Accordingly, there remains an urgent need in the art for improved therapeutic binding antagonists or antibodies, as well as methods for treating cancer using such agents.

### SUMMARY OF THE INVENTION

The present invention provides bispecific or trispecific antibodies comprising an LRRC15 antigen-binding domain. Also provided are methods of treating diseases such as cancer using the antibodies and antibody-drug conjugates of the present invention, as well as pharmaceutical compositions and articles of manufacture thereof.

In the first aspect of the present invention, it provides a multispecific antibody, which comprises:
a first targeting domain comprising one or more LRRC15 antigen binding domains;
a second targeting domain, which comprises one or more CD40 antigen-binding domains, or one or more CD3 antigen-binding domains;
optionally, a third targeting domain, wherein the third targeting domain binds to a target protein selected from the group consisting of PD-1, Claudin18.2 (CLDN18.2), and FAP; and/or a fourth targeting domain that binds to TGF-β.

In another preferred embodiment, the targeting domain is in the form of a single chain Fv (scFv), a Fab fragment, a single domain antibody (sdAb), a fragment variable (Fv) heterodimer, a TriFab, or a combination thereof.

In another preferred embodiment, the LRRC15 antigen binding domain comprises three heavy chain variable region CDRs (HCDRs), and three light chain variable region CDRs (LCDRs) selected from the group consisting of:

| Antibody Name | SEQ ID NO of HCDR1 | SEQ ID NO of HCDR2 | SEQ ID NO of HCDR3 | SEQ ID NO of LCDR1 | SEQ ID NO of LCDR2 | SEQ ID NO of LCDR3 |
|---|---|---|---|---|---|---|
| L15-06/ L15-06C | 33 | 34 | 35 | 45 | 46 | 47 |
| L15-08 | 36 | 37 | 38 | 45 | 48 | 49 |
| L15-03 | 39 | 40 | 41 | 50 | 51 | 52 |
| L15-07 | 42 | 43 | 44 | 50 | 51 | 53 |

In another preferred embodiment, the LRRC15 antigen binding domain comprises the following three heavy chain variable region CDRs:
HCDR1 having an amino acid sequence set forth in SEQ ID NO: 33;
HCDR2 having an amino acid sequence set forth in SEQ ID NO: 34; and
HCDR3 having an amino acid sequence set forth in SEQ ID NO: 35;
and, the following three light chain variable region CDRs:
LCDR1 having an amino acid sequence set forth in SEQ ID NO: 45;
LCDR2 having an amino acid sequence set forth in SEQ ID NO: 46; and
LCDR3 having an amino acid sequence set forth in SEQ ID NO: 47.

In another preferred embodiment, the LRRC15 antigen binding domain comprises a heavy chain variable region having at least 80% sequence identity to the amino acid sequence set forth in SEQ ID NO: 1, 2, 3, 4 or 5.

In another preferred embodiment, the LRRC15 antigen binding domain comprises a light chain variable region having at least 80% sequence identity to the amino acid sequence set forth in SEQ ID NO: 6, 7, 8, 9 or 10.

In another preferred embodiment, the LRRC15 antigen binding domain comprises a heavy chain variable region set forth in SEQ ID NO: 1, and a light chain variable region set forth in SEQ ID NO: 6.

In another preferred embodiment, the LRRC15 antigen binding domain comprises a heavy chain variable region set forth in SEQ ID NO: 2, and a light chain variable region set forth in SEQ ID NO: 7.

In another preferred embodiment, the LRRC15 antigen binding domain comprises a heavy chain variable region set forth in SEQ ID NO: 3, and a light chain variable region set forth in SEQ ID NO: 8.

In another preferred embodiment, the LRRC15 antigen binding domain comprises a heavy chain variable region set forth in SEQ ID NO: 4, and a light chain variable region set forth in SEQ ID NO: 9.

In another preferred embodiment, the LRRC15 antigen binding domain comprises a heavy chain variable region set forth in SEQ ID NO: 5, and a light chain variable region set forth in SEQ ID NO: 10.

In another preferred embodiment, the LRRC15 antigen binding domain is selected from the group consisting of: scFv, Fab, and a combination thereof.

In another preferred embodiment, the LRRC15 antigen binding domain is scFv.

In another preferred embodiment, the LRRC15 antigen binding domain is Fab, which comprises a heavy chain variable region set forth in SEQ ID NO: 1, 2, 3, 4 or 5, and a light chain variable region set forth in SEQ ID NO: 6, 7, 8, 9 or 10; and
a heavy chain constant region CH1 set forth in SEQ ID NO: 55, 56 or 64, and a light chain constant region CL set forth in SEQ ID NO: 66, 67 or 68.

In another preferred embodiment, the multispecific antibody further comprises an Fc fragment.

In another preferred embodiment, the Fc fragment is derived from IgG1 or IgG4.

In another preferred embodiment, the Fc fragment is an Fc fragment derived from IgG1, which has an amino acid sequence set forth in SEQ ID NO: 57 or 58.

In another preferred embodiment, the Fc fragment derived from IgG1 has mutations selected from the group consisting of:
N297A,
L234F/L235E/P331S,
L234A/L235A/P329G,
Y349C/K370E/K409D/K439E,
S354C/D356K/E357K/D399K,
S354C/T366W,
Y349C/T366S/L368A/Y407V,
L234F/L235E/P331S/Y349C/K370E/K409D/K439E,
L234F/L235E/P331S/S354C/D356K/E357K/D399K,
L234F/L235E/P331S/S354C/T366W,
L234F/L235E/P331S/Y349C/T366S/L368A/Y407V,
L234A/L235A/P329G/Y349C/K370E/K409D/K439E,
L234A/L235A/P329G/S354C/D356K/E357K/D399K,
L234A/L235A/P329G/S354C/T366W,
L234A/L235A/P329G/Y349C/T366S/L368A/Y407V,
L234F/L235E/P331S/S354C/T366W,
L234F/L235E/P331S/Y349C/T366S/L368A/Y407V,
N297A/Y349C/K370E/K409D/K439E,
N297A/S354C/D356K/E357K/D399K,
N297A/S354C/T366W,
N297A/Y349C/T366S/L368A/Y407V;
C220S/L234A/L235A/P329G/S354C/D356K/E357K/D399K,
C220S/L234A/L235A/P329G/Y349C/K370E/K409D/K439E; and/or
S267E,
S267E/G236D,
S267E/S239D,
S267E/L328F.

In another preferred embodiment, the Fc fragment has an amino acid sequence as shown in any one of SEQ ID NOs: 59-62, or SEQ ID NOs: 96-98.

In another preferred embodiment, the Fc fragment is an Fc fragment derived from IgG4, which has an amino acid sequence as shown in SEQ ID NO: 65.

In another preferred embodiment, the Fc fragment derived from IgG4 has mutations selected from the group consisting of:
Y349C/K370E/R409D/K439E,
S354C/E356K/E357K/D399K,
S354C/T366W,
Y349C/T366S/L368A/Y407V; and/or
S228P/S267E,
S228P/S267E/G236D,
S228P/S267E/S239D,
S228P/S267E/L328F.

In another preferred embodiment, the multispecific antibody is a bispecific, trispecific, or tetraspecific antibody.

In another preferred embodiment, the multispecific antibody is a bispecific antibody.

In another preferred embodiment, the bispecific antibody comprises:
a first targeting domain, which comprises one or more LRRC15 antigen-binding domains; and a second targeting domain, which comprises one or more CD40 antigen-binding domains.

In another preferred embodiment, the bispecific antibody comprises:
a first targeting domain, which comprises one or more LRRC15 antigen-binding domains; and a second targeting domain, which comprises one or more CD3 antigen-binding domains.

In another preferred embodiment, the multispecific antibody is a trispecific antibody.

In another preferred embodiment, the trispecific antibody comprises:
a first targeting domain, which comprises one or more LRRC15 antigen-binding domains;
a second targeting domain, which comprises one or more CD40 antigen-binding domains; and
a third targeting domain, which is a PD-1 antigen-binding domain.

In another preferred embodiment, the trispecific antibody comprises:
a first targeting domain, which comprises one or more LRRC15 antigen-binding domains;
a second targeting domain, which comprises one or more CD40 antigen-binding domains; and
a third targeting domain, which is a Claudin18.2 antigen-binding domain.

In another preferred embodiment, the trispecific antibody comprises:
a first targeting domain, which comprises one or more LRRC15 antigen-binding domains;
a second targeting domain, which comprises one or more CD3 antigen-binding domains; and
a third targeting domain, which is an FAP antigen-binding domain.

In another preferred embodiment, the antibody is a tetraspecific antibody.

In another preferred embodiment, the tetraspecific antibody comprises:
a first targeting domain, which comprises one or more LRRC15 antigen-binding domains;
a second targeting domain, which comprises one or more CD3 antigen-binding domains;
a third targeting domain, which is an FAP antigen-binding domain; and
a fourth targeting domain, which is a TGF-β antigen-binding domain.

In another preferred embodiment, the CD40 antigen-binding domain comprises a heavy chain variable region having at least 80% sequence identity to the amino acid sequence set forth in SEQ ID NO: 11, and a light chain variable region having at least 80% sequence identity to the amino acid sequence set forth in SEQ ID NO: 12.

In another preferred embodiment, the CD40 antigen-binding domain comprises a heavy chain variable region of the amino acid sequence set forth in any one of SEQ ID NOs: 13-23 and 114-116, and a light chain variable region of the amino acid sequence set forth in SEQ ID NO: 12.

In another preferred embodiment, the CD40 antigen-binding domain comprises a heavy chain variable region of the amino acid sequence set forth in SEQ ID NO: 11, and a light chain variable region of the amino-acid sequence set forth in any one of SEQ ID NOs: 24-26.

In another preferred embodiment, the CD40 antigen-binding domain comprises a heavy chain variable region of the amino acid sequence set forth in SEQ ID NO: 15, and a light chain variable region of the amino acid sequence set forth in SEQ ID NO: 12.

In another preferred embodiment, the CD40 antigen-binding domain comprises a heavy chain variable region of the amino acid sequence set forth in SEQ ID NO: 27, and a light chain variable region having at least 80% sequence identity to the amino acid sequence set forth in SEQ ID NO: 28.

In another preferred embodiment, the CD40 antigen-binding domain is selected from the group consisting of scFv, Fab, and combinations thereof.

In another preferred embodiment, the CD3 antigen-binding domain comprises a heavy chain variable region having at least 80% sequence identity to the amino acid sequence set forth in any one of SEQ ID NOs: 82-84 and 117, and a light chain variable region having at least 80% sequence identity to the amino acid sequence set forth in SEQ ID NO: 85.

In another preferred embodiment, the CD3 antigen-binding domain is selected from the group consisting of scFv, Fab, and combinations thereof.

In another preferred embodiment, the PD-1 antigen-binding domain comprises a heavy chain variable region having at least 80% sequence identity to the amino acid sequence set forth in SEQ ID NO: 29, and a light chain variable region having at least 80% sequence identity to the amino acid sequence set forth in SEQ ID NO: 30.

In another preferred embodiment, the PD-1 antigen-binding domain is selected from the group consisting of scFv, Fab, and combinations thereof.

In another preferred embodiment, the Claudin18.2 antigen-binding domain comprises a heavy chain variable region having at least 80% sequence identity to the amino acid sequence set forth in SEQ ID NO: 31, and a light chain variable region having at least 80% sequence identity to the amino acid sequence set forth in SEQ ID NO: 32.

In another preferred embodiment, the Claudin18.2 antigen-binding domain is selected from the group consisting of scFv, Fab, and combinations thereof.

In another preferred embodiment, the FAP antigen-binding domain comprises:
a heavy chain variable region having at least 80% sequence identity to the amino acid sequence set forth in SEQ ID NO: 86, and a light chain variable region having at least 80% sequence identity to the amino acid sequence set forth in SEQ ID NO: 87; or a heavy chain variable region having at least 80% sequence identity to the amino acid sequence set forth in SEQ ID NO: 88, and a light chain variable region having at least 80% sequence identity to the amino-acid sequence set forth in SEQ ID NO: 89; or a heavy chain variable region having at least 80% sequence identity to the amino acid sequence set forth in SEQ ID NO: 90, and a light chain variable region having at least 80% sequence identity to the amino acid sequence set forth in SEQ ID NO: 91; or a heavy chain variable region having at least 80% sequence identity to the amino-acid sequence set forth in SEQ ID NO: 92, and a light chain variable region having at least 80% sequence identity to the amino acid sequence set forth in SEQ ID NO: 93.

In another preferred embodiment, the FAP antigen-binding domain is selected from the group consisting of scFv, Fab, and combinations thereof.

In another preferred embodiment, the TGF-β antigen-binding domain is the receptor for TGF-β: TGF-βRII.

In another preferred embodiment, the TGF-β antigen-binding domain comprises an amino acid sequence having at least 80% sequence identity to the amino acid sequence set forth in SEQ ID NO: 94 or 95.

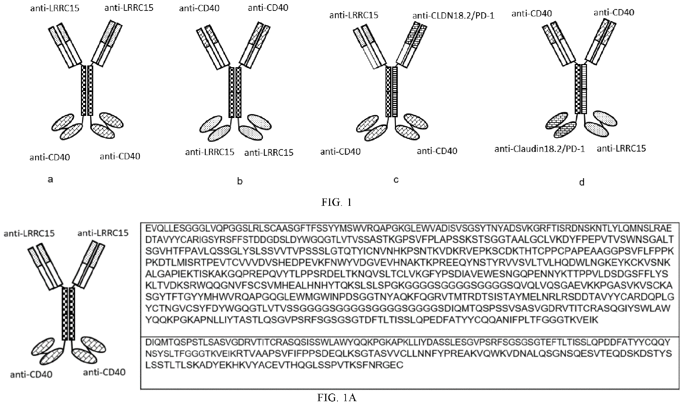

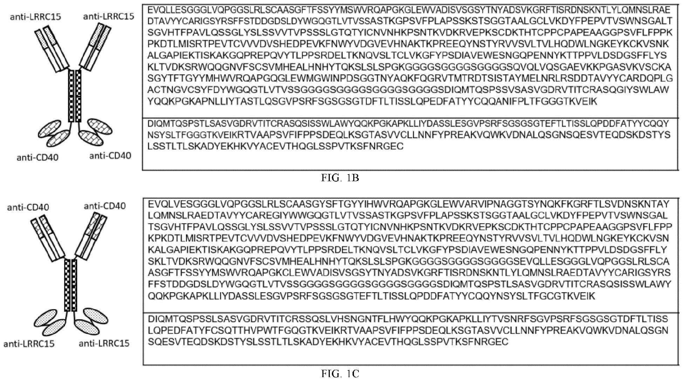

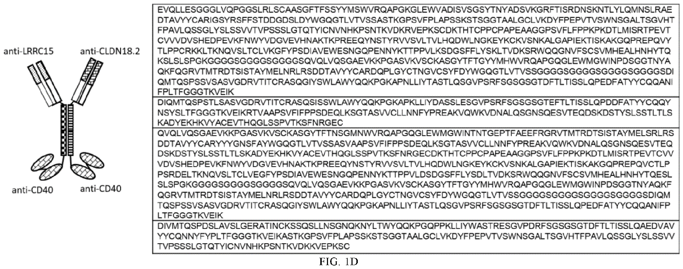

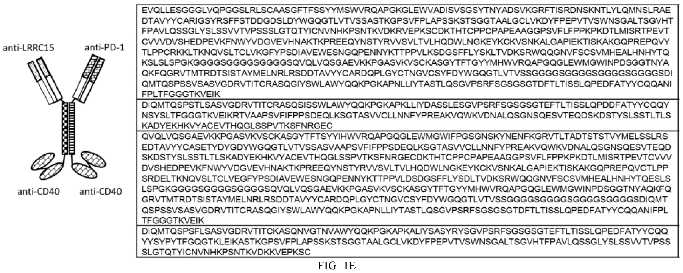

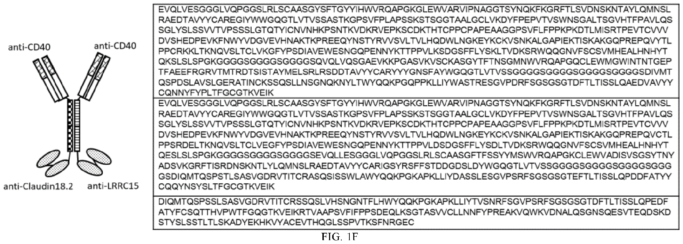

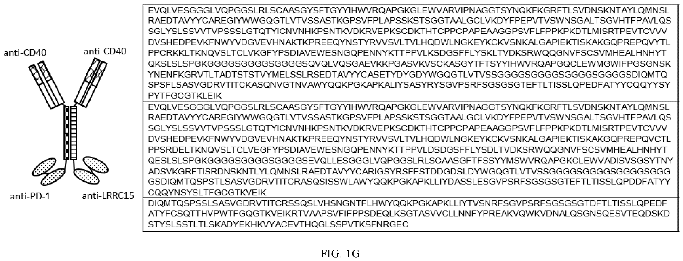

In another preferred embodiment, the multispecific antibody has a structure represented by Formula I below (panel a in FIG. 1): 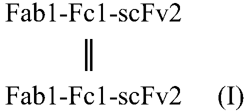
wherein each "-" is independently a peptide bond or a linker peptide; and "||" represents a linkage between peptide chains;
Fab1 is a first targeting domain, and the Fab1 is an anti-LRRC15 Fab;
scFv2 is a second targeting domain, and the scFv2 is an anti-CD40 scFv;
Fc1 is an Fc fragment.

In another preferred embodiment, the "||||" is a disulfide bond.

In another preferred embodiment, the anti-LRRC15 Fab comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the anti-LRRC15 Fab comprises a heavy chain variable region set forth in SEQ ID NO: 1, and a light chain variable region set forth in SEQ ID NO: 5.

In another preferred embodiment, the anti-CD40 scFv comprises a heavy chain variable region set forth in any one of SEQ ID NOs: 11, 13-23 and 114-116, and a light chain variable region set forth in any one of SEQ ID NOs: 12, 24-26; or
a heavy chain variable region set forth in SEQ ID NO: 27, and a light chain variable region set forth in SEQ ID NO: 28.

In another preferred embodiment, the anti-CD40 scFv comprises a heavy chain variable region set forth in SEQ ID NO: 15, and a light chain variable region set forth in SEQ ID NO: 12.

In another preferred embodiment, the anti-CD40 scFv comprises a heavy chain variable region set forth in SEQ ID NO: 11, and a light chain variable region set forth in SEQ ID NO: 12.

In another preferred embodiment, the anti-CD40 scFv comprises a heavy chain variable region set forth in SEQ ID NO: 19, and a light chain variable region set forth in SEQ ID NO: 12.

In another preferred embodiment, the anti-CD40 scFv comprises a heavy chain variable region set forth in SEQ ID NO: 115, and a light chain variable region set forth in SEQ ID NO: 12.

In another preferred embodiment, the Fc fragment is derived from IgG1 or IgG4.

In another preferred embodiment, the Fc fragment is derived from IgG1.

In another preferred embodiment, the Fc fragment has the amino acid sequence set forth in SEQ ID NO: 59.

In another preferred embodiment, the amino acid sequence of "HC1(heavy chain)-Fc1-scFv2" in "Fab1-Fc1-scFv2" is set forth in SEQ ID NO: 69, and the amino acid sequence of "LC1(light chain)" is set forth in SEQ ID NO: 70.

In another preferred embodiment, the amino acid sequence of "HC1(heavy chain)-Fc1-scFv1" in "Fab1-Fc1-scFv1" is set forth in SEQ ID NO: 71, and the amino acid sequence of "LC1(light chain)" is set forth in SEQ ID NO: 70.

In another preferred embodiment, the amino acid sequence of "HC1(heavy chain)-Fc1-scFv1" in "Fab1-Fc1-scFv1" is set forth in SEQ ID NO: 118, and the amino acid sequence of "LC1(light chain)" is set forth in SEQ ID NO: 70.

In another preferred embodiment, the amino acid sequence of "HC1(heavy chain)-Fc1-scFv1" in "Fab1-Fc1-scFv1" is set forth in SEQ ID NO: 119, and the amino acid sequence of "LC1(light chain)" is set forth in SEQ ID NO: 70.

In another preferred embodiment, the multispecific antibody has a structure represented by Formula II below (panel b in FIG. 1): 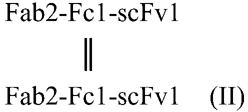
wherein each "-" is independently a peptide bond or a linker peptide; and "||" represents a linkage between peptide chains;
scFv1 is a first targeting domain, and the scFv1 is an anti-LRRC15 scFv;
Fab2 is a second targeting domain, and the Fab2 is an anti-CD40 Fab;
Fc1 is an Fc fragment.

In another preferred embodiment, the "||" is a disulfide bond.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD40 Fab comprises a heavy chain variable region set forth in any one of SEQ ID NOs: 11, 13-23 and 114-116, and a light chain variable region set forth in any one of SEQ ID NO: 12, 24-26; or
a heavy chain variable region set forth in SEQ ID NO: 27, and a light chain variable region set forth in SEQ ID NO: 28.

In another preferred embodiment, the anti-CD40 Fab comprises a heavy chain variable region set forth in SEQ ID NO: 27, and a light chain variable region set forth in SEQ ID NO: 28.

In another preferred embodiment, the Fc fragment is derived from IgG1 or IgG4.

In another preferred embodiment, the Fc fragment is derived from IgG1.

In another preferred embodiment, the Fc fragment has the amino acid sequence set forth in SEQ ID NO: 59.

In another preferred embodiment, the amino acid sequence of "HC2(heavy chain)-Fc1-scFv1" in "Fab2-Fc1-scFv1" is set forth in SEQ ID NO: 72, and the amino acid sequence of "LC2(light chain)" is set forth in SEQ ID NO: 73.

In another preferred embodiment, the multispecific antibody has a structure represented by Formula III below (panel c in FIG. 1): 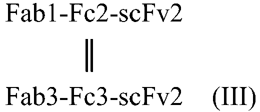
wherein each "-" is independently a peptide bond or a linker peptide; and "||" represents a linkage between peptide chains;
Fab1 is a first targeting domain, and the Fab1 is an anti-LRRC15 Fab;
scFv2 is a second targeting domain, and the scFv2 is an anti-CD40 scFv;
Fab3 is a third targeting domain, and the Fab3 is an anti-PD-1 Fab or an anti-CLDN18.2 Fab;
Fc2 and Fc3 are Fc fragments.

In another preferred embodiment, the anti-LRRC15 Fab comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 Fab comprises a heavy chain variable region set forth in SEQ ID NO: 1, and a light chain variable region set forth in SEQ ID NO: 5.

In another preferred embodiment, the anti-CD40 scFv comprises a heavy chain variable region set forth in any one of SEQ ID NOs: 11, 13-23 and 114-116, and a light chain variable region set forth in any one of SEQ ID NOs: 12, 24-26; or
a heavy chain variable region set forth in SEQ ID NO: 27, and a light chain variable region set forth in SEQ ID NO: 28.

In another preferred embodiment, the anti-CD40 scFv comprises a heavy chain variable region set forth in SEQ ID NO: 11, and a light chain variable region set forth in SEQ ID NO: 12.

In another preferred embodiment, the anti-PD-1 Fab comprises a heavy chain variable region set forth in SEQ ID NO: 29, and a light chain variable region set forth in SEQ ID NO: 30.

In another preferred embodiment, the anti-CLDN18.2 Fab comprises a heavy chain variable region set forth in SEQ ID NO: 31, and a light chain variable region set forth in SEQ ID NO: 32.

In another preferred embodiment, the Fc fragment is derived from IgG1 or IgG4.

In another preferred embodiment, the Fc fragment is derived from IgG1.

In another preferred embodiment, the Fc fragment has the amino acid sequence set forth in SEQ ID NO: 60 or 62.

In another preferred embodiment, the amino acid sequence of "HC1(heavy chain)-Fc2-scFv2" in "Fab1-Fc2-scFv2" is set forth in SEQ ID NO: 74, and the amino acid sequence of "LC1(light chain)" is set forth in SEQ ID NO: 70;
the amino acid sequence of "HC3(heavy chain)-Fc3-scFv2" in "Fab3-Fc3-scFv2" is set forth in SEQ ID NO: 75, and the amino acid sequence of "LC3(light chain)" is set forth in SEQ ID NO: 76.

In another preferred embodiment, the amino acid sequence of "HC1(heavy chain)-Fc2-scFv2" in "Fab1-Fc2-scFv2" is set forth in SEQ ID NO: 74, and the amino acid sequence of "LC1(light chain)" is set forth in SEQ ID NO: 70;
the amino acid sequence of "HC3(heavy chain)-Fc3-scFv2" in "Fab3-Fc3-scFv2" is set forth in SEQ ID NO: 77, and the amino acid sequence of "LC3(light chain)" is set forth in SEQ ID NO: 78.

In another preferred embodiment, the multispecific antibody has a structure represented by Formula IV below (panel d in FIG. 1): 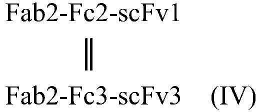
wherein each "-" is independently a peptide bond or a linker peptide; and "||" represents a linkage between peptide chains;
scFv1 is a first targeting domain, and the scFv1 is an anti-LRRC15 scFv;
Fab2 is a second targeting domain, and the Fab2 is an anti-CD40 Fab;
scFv3 is a third targeting domain, and the scFv3 is an anti-PD-1 scFv or an anti-CLDN18.2 scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD40 Fab comprises a heavy chain variable region set forth in any one of SEQ ID NOs: 11, 13-23 and 114-116, and a light chain variable region set forth in any one of SEQ ID NOs: 12, 24-26; or
a heavy chain variable region set forth in SEQ ID NO: 27, and a light chain variable region set forth in SEQ ID NO: 28.

In another preferred embodiment, the anti-CD40 Fab comprises a heavy chain variable region set forth in SEQ ID NO: 27, and a light chain variable region set forth in SEQ ID NO: 28.

In another preferred embodiment, the anti-PD-1 scFv comprises a heavy chain variable region set forth in SEQ ID NO: 29, and a light chain variable region set forth in SEQ ID NO: 30.

In another preferred embodiment, the anti-CLDN18.2 scFv comprises a heavy chain variable region set forth in SEQ ID NO: 31, and a light chain variable region set forth in SEQ ID NO: 32.

In another preferred embodiment, the anti-PD-1 scFv and the anti-CLDN18.2 scFv comprise cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 60 or 61.

In another preferred embodiment, the amino acid sequence of "HC2(heavy chain)-Fc2-scFv1" in "Fab2-Fc2-scFv1" is set forth in SEQ ID NO: 79, and the amino acid sequence of "LC2(light chain)" is set forth in SEQ ID NO: 73;
the amino acid sequence of "HC2(heavy chain)-Fc3-scFv3" in "Fab2-Fc3-scFv3" is set forth in SEQ ID NO: 80, and the amino acid sequence of "LC2(light chain)" is set forth in SEQ ID NO: 73.

In another preferred embodiment, the amino acid sequence of "HC2(heavy chain)-Fc2-scFv1" in "Fab2-Fc2-scFv1" is set forth in SEQ ID NO: 79, and the amino acid sequence of "LC2(light chain)" is set forth in SEQ ID NO: 73;
the amino acid sequence of "HC2(heavy chain)-Fc3-scFv3" in "Fab2-Fc3-scFv3" is set forth in SEQ ID NO: 81, and the amino acid sequence of "LC2(light chain)" is set forth in SEQ ID NO: 73.

In another preferred embodiment, the multispecific antibody has a structure represented by Formula V below (panel a inFIG. 2): 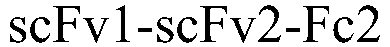 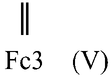
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv1-scFv2-Fc2" is set forth in SEQ ID NO: 99; and the amino acid sequence of Fc3 is set forth in SEQ ID NO: 98.

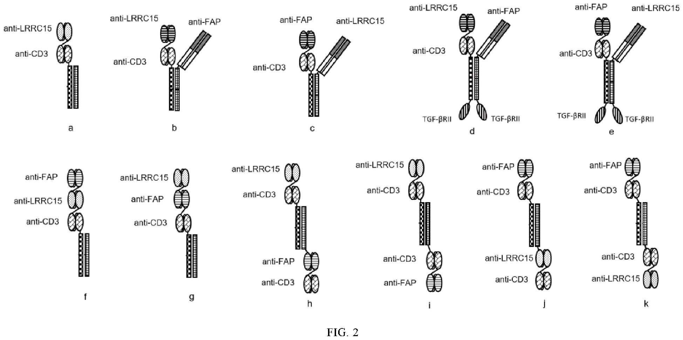

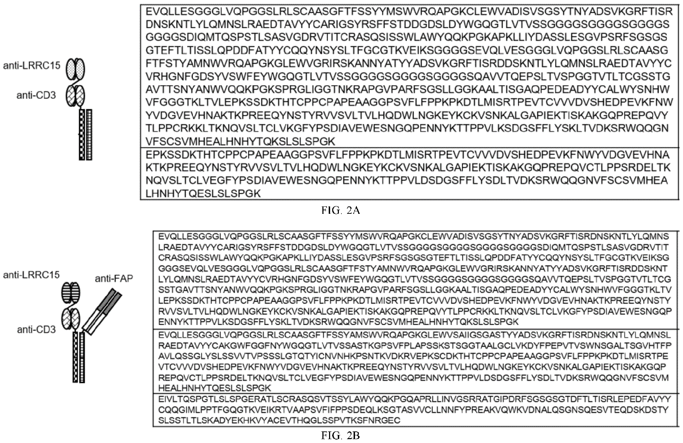

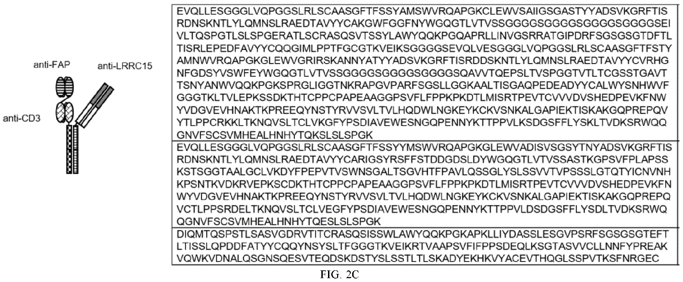

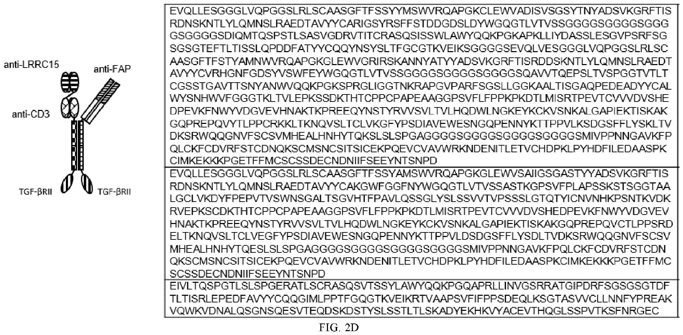

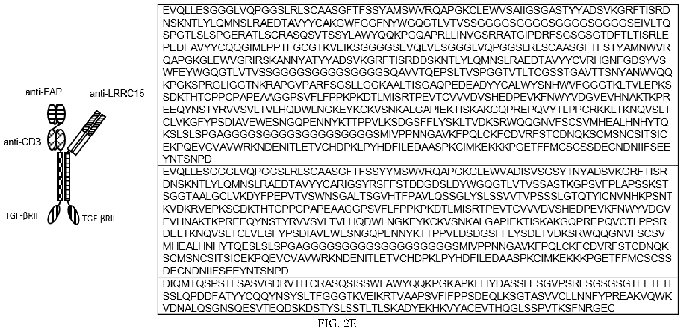

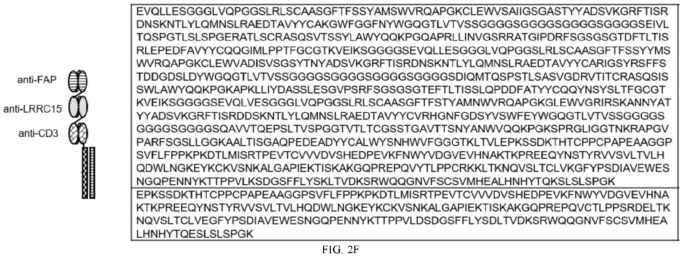

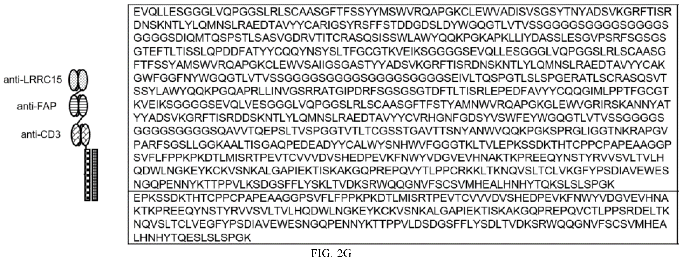

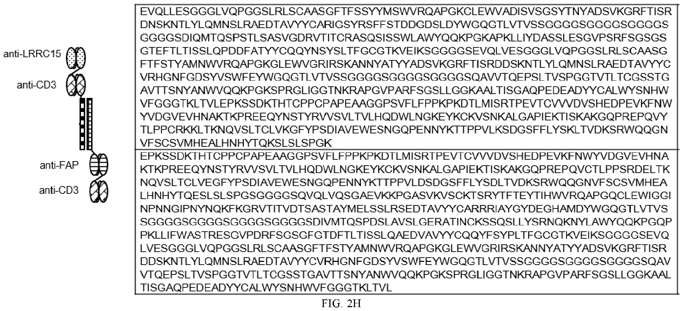

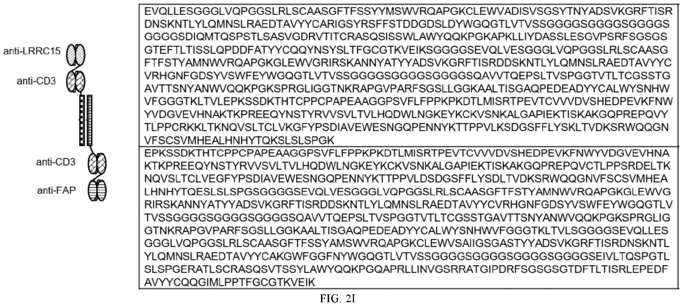

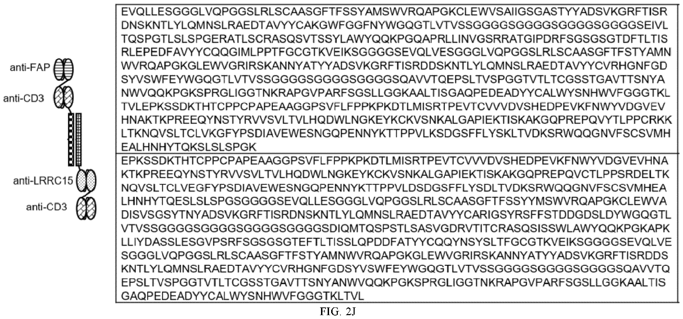

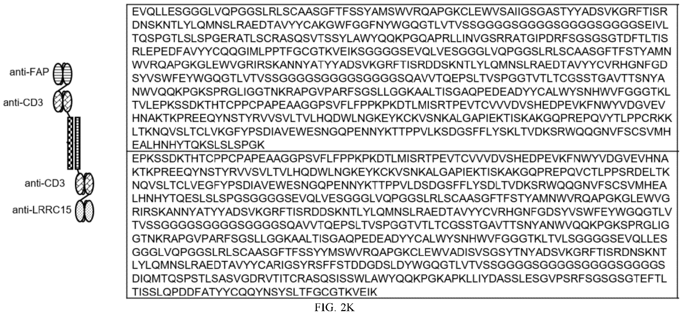

In another preferred embodiment, the multispecific antibody has a structure represented by Formula VI below (panel b in FIG. 2): 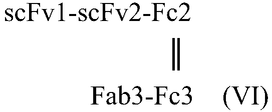
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
Fab3 is a third targeting domain, and the Fab3 is an anti-FAP Fab;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP Fab comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP Fab comprises a heavy chain variable region set forth in SEQ ID NO: 86, and a light chain variable region set forth in SEQ ID NO: 87.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 96 or 97.

In another preferred embodiment, the amino acid sequence of "scFv1-scFv2-Fc2" is set forth in SEQ ID NO: 99; the amino acid sequence of "HC3(heavy chain)-Fc3" in "Fab3-Fc3" is set forth in SEQ ID NO: 100, and the amino acid sequence of "LC3(light chain)" is set forth in SEQ ID NO: 101.

In another preferred embodiment, the multispecific antibody has a structure represented by Formula VI below (panel c in FIG. 2): 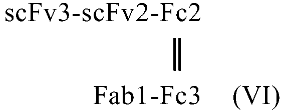
wherein each "-" is independently a peptide bond or a linker peptide; and "||" represents a linkage between peptide chains;
Fab1 is a first targeting domain, and the Fab1 is an anti-LRRC15 Fab;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 Fab comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 Fab comprises a heavy chain variable region set forth in SEQ ID NO: 1, and a light chain variable region set forth in SEQ ID NO: 6.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 96 or 97.

In another preferred embodiment, the amino acid sequence of "scFv3-scFv2-Fc2" is set forth in SEQ ID NO: 102; the amino acid sequence of "HC1(heavy chain)-Fc3" in "Fab1-Fc3" is set forth in SEQ ID NO: 103, and the amino acid sequence of "LC1(light chain)" is set forth in SEQ ID NO: 70.

In another preferred embodiment, the multispecific antibody has a structure represented by Formula VII below (panel d in FIG. 2): 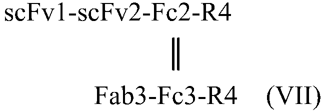
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
Fab3 is a third targeting domain, and the Fab3 is an anti-FAP Fab;
R4 is a fourth targeting domain, and the R4 is TGF-βRII;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NO: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP Fab comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP Fab comprises a heavy chain variable region set forth in SEQ ID NO: 86, and a light chain variable region set forth in SEQ ID NO: 87.

In another preferred embodiment, the amino acid sequence of TGF-βRII is set forth in SEQ ID NO: 94 or 95.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 96 or 97.

In another preferred embodiment, the amino acid sequence of "scFv1-scFv2-Fc2-R4" is set forth in SEQ ID NO: 104; the amino acid sequence of "HC3(heavy chain)-Fc3-R4" in "Fab3-Fc3-R4" is set forth in SEQ ID NO: 105, and the amino acid sequence of "LC3(light chain)" is set forth in SEQ ID NO: 101.

In another preferred embodiment, the multispecific antibody has a structure represented by Formula VIII below (panel e in FIG. 2): 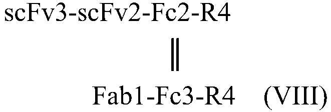
wherein each "-" is independently a peptide bond or a linker peptide; and "||" represents a linkage between peptide chains;
Fab1 is a first targeting domain, and the Fab1 is an anti-LRRC15 Fab;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 Fab comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 Fab comprises a heavy chain variable region set forth in SEQ ID NO: 1, and a light chain variable region set forth in SEQ ID NO: 6.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the amino acid sequence of TGF-βRII is set forth in SEQ ID NO: 94 or 95.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 96 or 97.

In another preferred embodiment, the amino acid sequence of "scFv3-scFv2-Fc2-R4" is set forth in SEQ ID NO: 106; the amino acid sequence of "HC1(heavy chain)-Fc3-R4" in "Fab1-Fc3-R4" is set forth in SEQ ID NO: 107, and the amino acid sequence of "LC1(light chain)" is set forth in SEQ ID NO: 70.

In another preferred embodiment, the multispecific antibody has a structure represented by Formula IX below (panel f in FIG. 2): 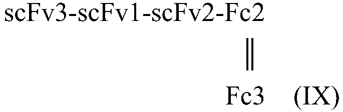
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv3-scFv1-scFv2-Fc2" is set forth in SEQ ID NO: 108; and the amino acid sequence of Fc3 is set forth in SEQ ID NO: 98.

In another preferred embodiment, the multispecific antibody has a structure represented by Formula X below (panel g in FIG. 2): 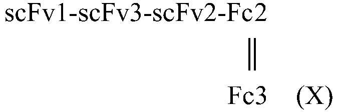
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv1-scFv3-scFv2-Fc2" is set forth in SEQ ID NO: 109; and the amino acid sequence of Fc3 is set forth in SEQ ID NO: 98.

In another preferred embodiment, the multispecific antibody has a structure represented by Formula XI below (panel h in FIG. 2): 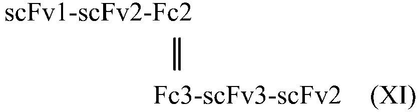
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises a heavy chain variable region set forth in SEQ ID NO: 92, and a light chain variable region set forth in SEQ ID NO: 93.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv1-scFv2-Fc2" is set forth in SEQ ID NO: 99; and the amino acid sequence of "Fc3-scFv3-scFv2" is set forth in SEQ ID NO: 110.

In another preferred embodiment, the multispecific antibody has a structure represented by Formula XII below (panel i in FIG. 2): 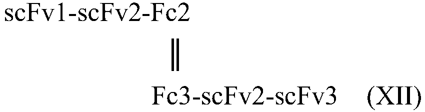
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv1-scFv2-Fc2" is set forth in SEQ ID NO: 99; and the amino acid sequence of "Fc3-scFv2-scFv3" is set forth in SEQ ID NO: 111.

In another preferred embodiment, the multispecific antibody has a structure represented by Formula XIII or Formula XIV below (panels j and k in FIG. 2): 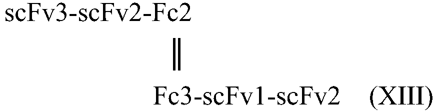 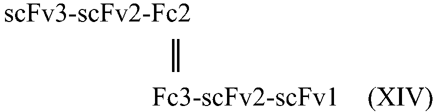
wherein each "-" is independently a peptide bond or a linker peptide; and "||" represents a linkage between peptide chains;
scFv1 is a first targeting domain, and the scFv1 is an anti-LRRC15 scFv;
scFv2 is a second targeting domain, and the scFv2 is an anti-CD3 scFv;
scFv3 is a third targeting domain, and the scFv3 is an anti-FAP scFv;
Fc2 and Fc3 are Fc fragments.

In another preferred embodiment, the anti-LRRC15 scFv comprises a heavy chain variable region set forth in any one of SEQ ID NO: 1, 2, 3, 4 and 5, and a light chain variable region set forth in any one of SEQ ID NO: 6, 7, 8, 9 and 10.

In another preferred embodiment, the LRRC15 scFv comprises a heavy chain variable region set forth in SEQ ID NO: 5, and a light chain variable region set forth in SEQ ID NO: 10.

In another preferred embodiment, the anti-CD3 scFv comprises a heavy chain variable region set forth in any one of SEQ ID NOs: 82-84 and 117, and a light chain variable region set forth in SEQ ID NO: 85.

In another preferred embodiment, the anti-CD3 scFv comprises a heavy chain variable region set forth in SEQ ID NO: 83, and a light chain variable region set forth in SEQ ID NO: 85.

In another preferred embodiment, the anti-FAP scFv comprises a heavy chain variable region set forth in SEQ ID NO: 86, and a light chain variable region set forth in SEQ ID NO: 87; or a heavy chain variable region set forth in SEQ ID NO: 88, and a light chain variable region set forth in SEQ ID NO: 89; or a heavy chain variable region set forth in SEQ ID NO: 90, and a light chain variable region set forth in SEQ ID NO: 91; or a heavy chain variable region set forth in SEQ ID NO: 92, and a light chain variable region set forth in SEQ ID NO: 93.

In another preferred embodiment, the anti-FAP scFv comprises a heavy chain variable region set forth in SEQ ID NO: 86, and a light chain variable region set forth in SEQ ID NO: 87.

In another preferred embodiment, the anti-FAP scFv comprises cysteine (C) mutations at VH44 and VL100 for improving the stability of the multispecific antibody.

In another preferred embodiment, the Fc fragment is derived from IgG1 or IgG4.

In another preferred embodiment, the Fc fragment is derived from IgG1.

In another preferred embodiment, the Fc fragment has an amino acid sequence set forth in SEQ ID NO: 97 or 98.

In another preferred embodiment, the amino acid sequence of "scFv3-scFv2-Fc2" is set forth in SEQ ID NO: 102; and the amino acid sequence of "Fc3-scFv1-scFv2" is set forth in SEQ ID NO: 112.

In another preferred embodiment, the amino acid sequence of "scFv3-scFv2-Fc2" is set forth in SEQ ID NO: 102; and the amino acid sequence of "Fc3-scFv2-scFv1" is set forth in SEQ ID NO: 113.

In the second aspect of the present invention, it provides a polynucleotide encoding the multispecific antibody according to the first aspect of the present invention.

In the third aspect of the present invention, it provides an expression vector comprising the polynucleotide according to the second aspect of the present invention.

In another preferred embodiment, the expression vector includes a prokaryotic expression vector or a eukaryotic expression vector.

In the fourth aspect of the present invention, it provides a host cell comprising the expression vector according to the third aspect of the present invention, or the polynucleotide according to the second aspect of the present invention integrated into the genome thereof.

In another preferred embodiment, the host cell includes a prokaryotic cell or a eukaryotic cell.

In another preferred embodiment, the host cell is selected from the group consisting of: *Escherichia coli*, yeast cells, HEK 293T cells, CHO cells.

In the fifth aspect of the present invention, it provides a use of the multispecific antibody according to the first aspect of the present invention for preparing a medicament for treating cancer/tumor, and/or fibrotic disease.

In another preferred embodiment, the cancer/tumor is a cancer/tumor with high expression of LRRC15.

In another preferred embodiment, the cancer/tumor is a cancer/tumor with high expression of LRRC15 and PD-1.

In another preferred embodiment, the cancer/tumor is a cancer/tumor with high expression of LRRC15 and CLDN18.2.

In another preferred embodiment, the cancer/tumor is a cancer/tumor with high expression of LRRC15 and FAP.

In another preferred embodiment, the cancer/tumor includes solid tumors and hematological tumors.

In another preferred embodiment, the cancer/tumor is a solid tumor.

In another preferred embodiment, the cancer/tumor is an epithelial cancer or tumor.

In another preferred embodiment, the cancer/tumor is selected from the group consisting of breast cancer, head and neck tumor, lung cancer, pancreatic cancer, ovarian cancer, melanoma, malignant glioma, sarcoma, bone cancer, colon cancer, bronchial cancer, and combinations thereof.

In another preferred embodiment, the fibrotic disease includes fibrosis of multiple organs such as lung, liver, kidney, heart, brain, spleen, muscle and skin, as well as lesions associated with tumor-associated fibroblasts.

In the sixth aspect of the present invention, it provides an immunoconjugate comprising:
(i) the multispecific antibody according to the first aspect of the present invention; and
(ii) a coupling moiety selected from the group consisting of: a detectable label, a drug, a toxin, a cytokine, a radionuclide, and an enzyme.

In another preferred embodiment, the coupling moiety is selected from: fluorescent or luminescent labels, radioactive labels, MRI (magnetic resonance imaging) or CT (computed tomography) contrast agents, or enzymes capable of generating detectable products, radionuclides, biological toxins, cytokines (such as IL-2, etc.), antibodies, antibody Fc fragments, antibody scFv fragments, gold nanoparticles/nanorods, virus particles, liposomes, magnetic nanoparticles, prodrug activating enzymes (e.g., DT-diaphorase (DTD) or biphenyl hydrolase-like protein (BPHL)), chemotherapeutic agents (e.g., cisplatin) or any form of nanoparticles, etc.

In the seventh aspect of the present invention, it provides a pharmaceutical composition comprising: (a) the multispecific antibody according to the first aspect of the present invention, or the immunoconjugate according to the sixth aspect of the present invention; and (b) a pharmaceutically acceptable carrier.

In another preferred embodiment, the pharmaceutical composition is an injection.

In the eighth aspect of the present invention, it provides a use of the immunoconjugate according to the sixth aspect of the present invention for preparing a medicament for treating cancer/tumor, and/or fibrotic disease.

In another preferred embodiment, the cancer/tumor is a cancer/tumor with high expression of LRRC15.

In another preferred embodiment, the cancer/tumor is a cancer/tumor with high expression of LRRC15 and PD-1.

In another preferred embodiment, the cancer/tumor is a cancer/tumor with high expression of LRRC15 and CLDN18.2.

In another preferred embodiment, the cancer/tumor is a cancer/tumor with high expression of LRRC15 and FAP.

In another preferred embodiment, the cancer/tumor includes solid tumors and hematological tumors.

In another preferred embodiment, the cancer/tumor is a solid tumor.

In another preferred embodiment, the cancer/tumor is an epithelial cancer or tumor.

In another preferred embodiment, the cancer/tumor is selected from the group consisting of breast cancer, head and neck tumor, lung cancer, pancreatic cancer, ovarian cancer, melanoma, malignant glioma, sarcoma, bone cancer, colon cancer, bronchial cancer, and combinations thereof.

In another preferred embodiment, the fibrotic disease includes fibrosis of multiple organs such as lung, liver, kidney, heart, brain, spleen, muscle and skin, as well as lesions associated with tumor-associated fibroblasts.

In the ninth aspect of the present invention, it provides a method for treating cancer/tumor, and/or fibrotic disease, which comprises a step of administering the multispecific antibody according to the first aspect of the present invention, or the immunoconjugate according to the sixth aspect of the present invention to a subject in need thereof.

In another preferred embodiment, the subject in need thereof is a human or a non-human mammal.

In another preferred embodiment, the cancer/tumor is a cancer/tumor with high expression of LRRC15.

In another preferred embodiment, the cancer/tumor is a cancer/tumor with high expression of LRRC15 and PD-1.

In another preferred embodiment, the cancer/tumor is a cancer/tumor with high expression of LRRC15 and CLDN18.2.

In another preferred embodiment, the cancer/tumor is a cancer/tumor with high expression of LRRC15 and FAP.

In another preferred embodiment, the cancer/tumor includes solid tumors and hematological tumors.

In another preferred embodiment, the cancer/tumor is a solid tumor.

In another preferred embodiment, the cancer/tumor is an epithelial cancer or tumor.

In another preferred embodiment, the cancer/tumor is selected from the group consisting of breast cancer, head and neck tumor, lung cancer, pancreatic cancer, ovarian cancer, melanoma, malignant glioma, sarcoma, bone cancer, colon cancer, bronchial cancer, and combinations thereof.

In another preferred embodiment, the fibrotic disease includes fibrosis of multiple organs such as lung, liver, kidney, heart, brain, spleen, muscle and skin, as well as lesions associated with tumor-associated fibroblasts.

It should be understood that within the scope of the present invention, the above technical features of the present invention and the technical features specifically described below (such as examples) can be combined with each other to form new or preferred technical solutions. Due to space limitations, they are not listed here one by one.

### DESCRIPTION OF DRWAINGS

FIG. 1 shows two formats of bispecific antibody composed of an LRRC15 antigen binding domain and a CD40 antigen binding domain (a and b); and two formats of trispecific antibody composed of an LRRC15 antigen binding domain, a CD40 antigen binding domain, and a CLDN18.2 or PD-1 antigen binding domain (c and d).
FIG. 1A shows an exemplary bispecific antibody structure corresponding to panel a in FIG. 1, designated 1540s.
FIG. 1B shows another exemplary bispecific antibody structure corresponding to panel a in FIG. 1, designated 1540s-3.
FIG. 1C shows an exemplary bispecific antibody structure corresponding to panel b in FIG. 1, designated 40d15.
FIG. 1D shows an exemplary trispecific antibody corresponding to panel c in FIG. 1, designated 151840-L06.
FIG. 1E shows another exemplary trispecific antibody corresponding to panel c in FIG. 1, designated 15P140-L06.
FIG. 1F shows an exemplary trispecific antibody corresponding to panel d in FIG. 1, designated 40d1815.
FIG. 1G shows another exemplary trispecific antibody corresponding to panel d in FIG. 1, designated 40dP115.
FIG. 2 shows a format of bispecific antibody composed of an LRRC15 antigen binding domain and a CD3 antigen binding domain (a); formats of trispecific antibody composed of the foregoing two domains plus an FAP antigen binding domain (b, c, f, g, h, i, j, k); and formats of tetraspecific antibody composed of the foregoing three domains plus a TGF-β binding domain (d, e).
FIG. 2A shows an exemplary bispecific antibody structure corresponding to panel a in FIG. 2, designated 153.
FIG. 2B shows an exemplary trispecific antibody structure corresponding to panel b in FIG. 2, designated 315F.
FIG. 2C shows an exemplary trispecific antibody structure corresponding to panel c in FIG. 2, designated F315.
FIG. 2D shows an exemplary tetraspecific antibody structure corresponding to panel d in FIG. 2, designated 315Fb.
FIG. 2E shows an exemplary tetraspecific antibody structure corresponding to panel e in FIG. 2, designated F315b.
FIG. 2F shows an exemplary trispecific antibody structure corresponding to panel f in FIG. 2, designated F153.
FIG. 2G shows an exemplary trispecific antibody structure corresponding to panel g in FIG. 2, designated 15F3.
FIG. 2H shows an exemplary trispecific antibody structure corresponding to panel h in FIG. 2, designated 153F.
FIG. 2I shows an exemplary trispecific antibody structure corresponding to panel i in FIG. 2, designated 1533F.
FIG. 2J shows an exemplary trispecific antibody structure corresponding to panel j in FIG. 2, designated F3153.
FIG. 2K shows an exemplary trispecific antibody structure corresponding to panel k in FIG. 2, designated F3315.
FIG. 3A and FIG. 3B show detection results of DC cell activation induced by the constructed bispecific antibodies targeting LRRC15 and CD40.
FIG. 4A shows detection results of CD40 reporter gene signal activation induced by the constructed bispecific antibodies targeting LRRC15 and CD40.
FIG. 4B shows detection results of CD40 reporter gene signal activation induced by the constructed bispecific antibodies targeting LRRC15 and CD40.
FIG. 4C shows detection results of CD3 reporter gene signal activation induced by the constructed trispecific antibodies targeting LRRC15, FAP and CD3.
FIG. 5 shows detection results of cytokine release from PBMC stimulated by the bispecific antibodies targeting LRRC15 and CD40.
FIG. 6A shows results of tumor volume change in the KPC mouse model after administration of the bispecific antibodies targeting LRRC15 and CD40.
FIG. 6B shows results of body weight change in the KPC mouse model after administration of the bispecific antibodies targeting LRRC15 and CD40.
FIG. 7A shows results of tumor volume change in the MC38 mouse model after administration of control monoclonal antibody and the bispecific antibodies targeting LRRC15 and CD40, respectively.
FIG. 7B shows results of body weight change in the MC38 mouse model after administration of control monoclonal antibody and the bispecific antibodies targeting LRRC15 and CD40, respectively.
FIG. 7C shows the expression levels of ALT and AST in MC38 model mice induced by the control monoclonal antibody and the bispecific antibodies targeting LRRC15 and CD40 at 24 hours after administration.
FIG. 8 shows the effect of the bispecific antibodies targeting LRRC15 and CD40 on the body weight of cynomolgus monkeys.

### DETAILED DESCRIPTION

After extensive and in-depth research, the inventors of the present invention unexpectedly developed a class of multispecific antibodies comprising an LRRC15 antigen binding domain for the first time. The multispecific T cell engager contains a first targeting domain that targets leucine-rich repeat-containing 15 (LRRC15) highly expressed on the surface of tumor cells, wherein the first targeting domain comprises an LRRC15 antibody or an antigen-binding fragment thereof; meanwhile, the multispecific T cell engager further comprises a second targeting domain that binds to CD40 on the surface of antigen-presenting cells (APCs) or CD3 on the surface of T cells. Optionally, the multispecific T cell engager comprises a third targeting domain targeting PD-1, CLDN18.2 or FAP, and/or a fourth targeting domain targeting TGF-β. The LRRC15-related multispecific antibodies of the present invention can effectively activate T cells and mitigate stromal effects of tumors, and are capable of achieving effective treatment against tumors and tumor-associated fibrosis.

On this basis, the present invention is completed.

As used herein, the term "leucine-rich repeat-containing protein 15" or "LRRC15" refers to the protein encoded by the LRRC15 gene in humans. The leucine-rich repeat (LRR) domain is evolutionarily conserved in proteins associated with innate immunity. LRRC15 is expressed on stromal fibroblasts in many solid tumors (e.g., breast, head and neck, lung, pancreas) and directly on a subset of cancer cells of mesenchymal origin (e.g., sarcoma, melanoma, glioma), while most normal tissues show low or no expression.

As used herein, the terms "CD", "cluster of differentiation", or "cluster of differentiation molecules" refer to cell surface markers that can be used to identify and characterize leukocytes, such as CD40.

CD40 is a cell surface receptor that belongs to the tumor necrosis factor-R (TNF-R) family and is constitutively expressed on APCs such as B cells and dendritic cells. CD40L (CD154) is a type II transmembrane glycoprotein that can be induced and is restricted to cells of the hematopoietic system, such as platelets, granulocytes, and activated T/B/NK cells. CD40L binding to CD40 activates APCs, leading to upregulation of CD80, CD86 and other costimulatory molecules to stimulate CD8+ antigen-specific T cell responses.

The term "Fc fragment" or "Fc" refers to the portion of an antibody that does not have antigen binding activity but was originally observed to be easily crystallized, and hence was named Fc fragment (for fragment crystallizable). This fragment corresponds to the paired CH2 and CH3 domains, and is the portion of the antibody molecule that interacts with effector molecules and cells. The Fc fragments described herein can be derived from IgG1, IgG2, and IgG4 antibodies. For specific purposes, a particular IgG subclass may be preferred. For example, IgG1 is more effective than IgG2 and IgG4 in mediating ADCC and CDC. Therefore, IgG2 Fc may be preferred when effector function is not desired. However, molecules containing IgG2 Fc are generally more difficult to prepare and may be less stable than molecules containing IgG1 Fc. On the other hand, cross-binding to FcγRIIB is critical for enhancing costimulatory signals from CD40 or CD137, and IgG4 has an affinity about 2-fold higher than IgG1 and 10-fold higher than IgG2. Mutations such as S267E and L328F can further enhance the cross-binding of IgG Fc to FcγRIIB. Additionally, the effector function of an antibody can be increased or decreased by introducing one or more mutations into the Fc (see, e.g., Strohl, Curr. Opin. Biotech., 20:685-691, 2009).

As used herein, the term "antibody" or "immunoglobulin" refers to an approximately 150,000 dalton heterotetrameric protein with the same structural features, which is composed of two identical light chains (L) and two identical heavy chains (H). Each light chain is covalently linked to a heavy chain by a disulfide bond, and the number of disulfide bonds between the heavy chains of different immunoglobulin isotypes varies. Each heavy chain and light chain also has intra-chain disulfide bonds at regular intervals. One end of each heavy chain has a variable region (VH), followed by multiple constant regions. Each light chain has a variable region (VL) at one end and a constant region at the other end; the constant region of the light chain is opposite the first constant region of the heavy chain, and the variable region of the light chain is opposite the variable region of the heavy chain. Specific amino acid residues form an interface between the variable regions of the light and heavy chains.

As used herein, the term "variable" means that certain portions of the variable region of an antibody differ in sequence, and it gives rise to the binding and specificity of various specific antibodies for their specific antigens. However, variability is not evenly distributed throughout the antibody variable region. It is concentrated in three segments within the variable regions of the light and heavy chains called complementarity determining regions (CDRs) or hypervariable regions. The more conserved portions of the variable region are called framework regions (FRs). The variable regions of the natural heavy and light chains respectively contain four FR regions, which generally have a β-sheet configuration and are connected by three CDRs that form a connecting loop, forming partial β-sheet structures in some cases. The CDRs in each chain are closely clustered together through the FR regions and together with the CDRs of the other chain, forming the antigen-binding site of the antibody (see Kabat et al., NIH Publ. No. 91-3242, Vol. I, pages 647-669 (1991)). The constant regions do not directly participate in the binding of the antibody to the antigen, but they exhibit different effector functions, such as participating in antibody-dependent cytotoxicity of the antibody.

The antibodies of the present application can include, but are not limited to, polyclonal, monoclonal, monospecific, multispecific, bispecific, human, humanized, primateized, chimeric and single-chain antibodies. The antibodies disclosed herein can be from any animal origin, including birds and mammals. Preferably, the antibodies are human, mouse, donkey, rabbit, goat, guinea pig, camel, llama, horse or chicken antibodies.

The term "antibody fragment" or "antigen binding fragment" refers to a portion of an antibody, such as F(ab')2, F(ab)2, Fab', Fab, Fv, single-chain Fvs (scFv), single-chain antibodies, disulfide-linked Fvs (sdFv), fragments containing VL or VH domains, fragments produced by Fab expression libraries, and anti-idiotypic (anti-Id) antibodies. Regardless of structure, antibody fragments bind to the same antigen as the intact antibody recognizes. The term "antibody fragment" includes DARTs and diabodies. The term "antibody fragment" also includes any synthetic or genetically engineered protein that comprises an immunoglobulin variable region and acts like an antibody by forming a complex with a specific antigen. A "single-chain variable fragment" or "scFv" is a fusion protein of the variable regions of the heavy chain (VH) and light chain (VL) of an immunoglobulin. In some embodiments, the domain regions are connected by a short linker peptide of 10 to about 25 amino acids. The linker can be glycine-rich for flexibility and serine-rich or threonine-rich for solubility, and can be linked to the N-terminus of VH or the C-terminus of VL, or vice versa. Despite the removal of the constant region and the introduction of the linker, this protein still retains the specificity of the original immunoglobulin. For IgG, standard immunoglobulin molecules contain two identical light chain polypeptides of approximately 23,000 daltons and two identical heavy chain polypeptides of 53,000-70,000 daltons. The four chains are typically linked by disulfide bonds in a "Y" configuration, with the light chains connecting (bracketing) the heavy chains from the mouth of the "Y" and extending through the variable region.

As described above, the variable region allows the antibody to selectively recognize and specifically bind to an epitope on the antigen. That is, the VL domain and VH domain of the antibody or a subset of the complementarity determining regions (CDRs) of the antibody combine to form the variable region that defines the three-dimensional antigen binding site. This tetrameric antibody structure forms the antigen binding site present at the end of each arm of the Y configuration. More specifically, the antigen binding site is defined by the three CDRs (i.e., HCDR1, HCDR2, HCDR3, LCDR1, LCDR2, and LCDR3) on each of the VH and VL chains. In some cases, for example, certain immunoglobulin molecules are derived from camelid animal species or engineered based on camelid immunoglobulin. Alternatively, immunoglobulin molecules may be composed of only heavy chains without light chains or only light chains without heavy chains.

In naturally occurring antibodies, the six CDRs present in each antigen binding domain are short, non-contiguous amino acid sequences that are specifically positioned to form the "antigen binding domain" due to the three-dimensional configuration of the antibody in an aqueous environment. The remaining amino acids in the antigen binding domain, called "framework" region domains, exhibit less intermolecular variability. The framework regions adopt a predominantly β-sheet conformation, and the CDRs form loops that are connected and form a part of the β-sheet structure in some cases. Thus, the framework region acts as a scaffold that positions the CDRs in the correct orientation through interchain non-covalent interactions. The antigen binding domain formed by the positioned CDRs defines a surface that is complementary to the epitope on the immunoreactive antigen. This complementary surface promotes non-covalent binding of the antibody to its cognate epitope. Since they have been precisely defined, the ordinary skilled person in the art of domain structures can readily identify the amino acids comprising the CDRs and framework regions, respectively, for any given heavy or light chain variable region.

As used herein, the term "light chain constant region (CL)" encompasses the amino acid sequence CL (SEQ ID NOs: 66, 67, 68) derived from an antibody light chain. Preferably, the light chain constant region comprises at least one of the constant kappa domain or the constant lambda domain.

As used herein, the term "heavy chain constant region (CH)" encompasses the amino acid sequence derived from an immunoglobulin heavy chain. A polypeptide comprising a heavy chain constant region comprises at least one of the following: a CH1 domain, a hinge region (e.g., an upper, middle, and/or lower hinge region) domain, a CH2 domain, a CH3 domain, or a variant or fragment thereof. It is understood that the heavy chain constant regions can be modified such that their amino acid sequences differ from those of naturally occurring immunoglobulin molecules.

In one embodiment of the present invention, the prepared multispecific antibody adopts a CrossMab structure, in which partial amino acid sequences of the heavy chain CH1 domain and light chain CL domain are swapped to avoid chain mispairing. Such an exemplary structure comprises the CH1 domain set forth in SEQ ID NO: 56 and the CL domain set forth in SEQ ID NO: 68.

As used herein, a "variant" of an antibody, antibody fragment, or antibody domain refers to an antibody, antibody fragment, or antibody domain that: (1) has at least 80%, 85%, 90%, 95%, 96%, 97%, 98%, or 99% sequence identity to the original antibody, antibody fragment, or antibody domain, and (2) specifically binds to the same target as the original antibody, antibody fragment, or antibody domain specifically binds. It should be understood that in the case of the sequence identity being expressed in the form of "at least x% identical" or "at least x% identity", such embodiments include any and all numerical percentages equal to or greater than the lower limit. Further, it should be understood that in the presence of amino acid sequences in the present application, they should be construed as additionally disclosing or encompassing those having at least 80%, at least 85%, at least 90%, at least 95%, at least 96%, at least 97%, at least 98%, or at least 99% identity to said amino acid sequence.

The scope of the multi-specific molecules of the present invention includes a variety of compositions and methods, including: asymmetric IgG-like antibodies (e.g., trifunctional monoclonal antibodies (triomabs)/quadromas); knobs-into-holes antibodies; Cross monoclonal antibodies (Cross MAb); electrostatically matched antibodies; LUZ-Y; chain-exchange engineered domain (SEED) bodies; Fab-exchanged antibodies, symmetric IgG class antibodies; two-in-one antibodies; crosslinked monoclonal antibodies, mAb2; Cov X-body; dual variable domain (DVD)-Ig fusion proteins; IgG-like bispecific antibodies; Ts2Ab; BsAb; scFv/Fc fusions; dual (scFv)2-Fabs; F(ab)2 fusion proteins; bifunctional or Bis-Fab; Dock-and-Lock(DNL); Fab-Fv; scFv-based antibodies and bispecific antibody-based antibodies, such as bispecific T cell engagers (BiTEs); tandem bispecific antibodies (TandAb); DARTs; single-chain bispecific antibodies; TCR-like antibodies; human serum albumin scFv fusion proteins, COMBODIES and IgG/non-IgG fusion proteins.

As used herein, the phrase "multispecific antibody" refers to a molecule comprising at least two targeting domains having different binding specificities. In some embodiments, the multispecific inhibitor is a polypeptide comprising a scaffold and two or more immunoglobulin antigen binding domains targeting different antigens or epitopes. In some embodiments, the multispecific antibody is a bispecific antibody. In some other embodiments, the multispecific antibody is a trispecific antibody. In some other embodiments, the multispecific antibody is a tetraspecific antibody.

As used herein, the phrase "bispecific" refers to a molecule comprising at least two targeting domains having different binding specificities. Each targeting domain is capable of specifically binding to a target molecule and, upon binding to the target molecule, inhibits the biological function of the target molecule. In some embodiments, the bispecific T cell engager is a polymeric molecule having two or more peptides. In some embodiments, the targeting domain comprises the antigen binding domain or CDRs of an antibody. In some embodiments, the targeting domain comprises a ligand or fragment thereof that specifically binds to a target protein. In some embodiments, the bispecific inhibitor is a bispecific antibody.

The terms "bispecific antibody" and "bispecific T cell engager" can be used interchangeably herein to refer to an antibody that can specifically bind to two different antigens (or epitopes). In some embodiments, the bispecific antibody is a full-length antibody that binds one antigen (or epitope) on one of its two binding arms (one pair of HC/LC) and binds to a different antigen (or epitope) on its second arm (the other pair of HC/LC). In these embodiments, the bispecific antibody has two different antigen binding arms (both in terms of specificity and CDR sequence) and is monovalent for each antigen it binds to.

In some other embodiments, the bispecific antibody is a full-length antibody that can bind two different antigens (or epitopes) in each of its two binding arms (two pairs of HC/LC). In these embodiments, the bispecific antibody has two identical antigen binding arms, with the same specificity and the same CDR sequence, and is bivalent for each antigen it binds to.

The terms "trispecific antibody" and "trispecific molecule" can be used interchangeably herein to refer to a molecule comprising three targeting domains having three different binding specificities. Each targeting domain is capable of specifically binding to a target molecule and, upon binding to the target molecule, inhibits the biological function of the target molecule. In some embodiments, the trispecific antagonist is a polymeric molecule having two or more peptides. In some embodiments, the targeting domain comprises the antigen binding domain or CDRs of an antibody. In some embodiments, the targeting domain comprises a ligand or fragment thereof that specifically binds to a target protein.

In a preferred embodiment of the present invention, bispecific antibodies targeting LRRC15 and CD40 are constructed, having structures as shown in a-b of FIG. 1. An exemplary molecule of format a is shown in FIG. 2A or FIG. 2B, wherein the anti-LRRC15 Fab may be a Fab constructed using the VH and VL of any anti-LRRC15 antibody described in the present invention; and the anti-CD40 scFv may be an scFv constructed using the VH and VL of any anti-CD40 antibody described in the present invention. An exemplary molecule of format b is shown in FIG. 2C, wherein the anti-CD40 Fab may be a Fab constructed using the VH and VL of any anti-CD40 antibody described in the present invention; and the anti-LRRC15 scFv may be an scFv constructed using the VH and VL of any anti-LRRC15 antibody described in the present invention.

In another preferred embodiment of the present invention, trispecific antibodies targeting LRRC15, CD40 and CLDN18.2 are constructed, having structures as shown in c-d of FIG. 1. An exemplary molecule of format c is shown in FIG. 2D, wherein the anti-LRRC15 Fab may be a Fab constructed using the VH and VL of any anti-LRRC15 antibody described in the present invention, the anti-CD40 scFv may be an scFv constructed using the VH and VL of any anti-CD40 antibody described in the present invention, and the anti-CLDN18.2 Fab may be a Fab constructed using the VH and VL of any anti-CLDN18.2 antibody described in the present invention. An exemplary molecule of format d is shown in FIG. 2F, wherein the anti-LRRC15 scFv may be an scFv constructed using the VH and VL of any anti-LRRC15 antibody described in the present invention, the anti-CD40 Fab may be a Fab constructed using the VH and VL of any anti-CD40 antibody described in the present invention, and the anti-CLDN18.2 scFv may be an scFv constructed using the VH and VL of any anti-CLDN18.2 antibody described in the present invention.

In another preferred embodiment of the present invention, trispecific antibodies targeting LRRC15, CD40 and PD-1 are constructed, having structures depicted as c-d in FIG. 1. An exemplary molecule of format c is shown in FIG. 2E, in which the anti-LRRC15 Fab can be a Fab constructed with VH and VL of any anti-LRRC15 antibody described herein, the anti-CD40 scFv can be an scFv constructed with VH and VL of any anti-CD40 antibody described herein, and the anti-PD-1 Fab can be a Fab constructed with VH and VL of any anti-PD-1 antibody described herein. An exemplary molecule of format d is shown in FIG. 2G, in which the anti-LRRC15 scFv can be an scFv constructed with VH and VL of any anti-LRRC15 antibody described herein, the anti-CD40 Fab can be a Fab constructed with VH and VL of any anti-CD40 antibody described herein, and the anti-PD-1 scFv can be an scFv constructed with VH and VL of any anti-PD-1 antibody described herein.

In another preferred embodiment of the present invention, bispecific antibodies targeting LRRC15 and CD3 are constructed, having the structure shown as format a in FIG. 2.

In another preferred embodiment of the present invention, trispecific antibodies targeting LRRC15, CD3 and FAP are constructed, having structures shown as formats b, c, f, g, h, i, j and k in FIG. 2.

In another preferred embodiment of the present invention, tetraspecific antibodies targeting LRRC15, CD3, FAP and TGF-β are constructed, having structures shown as formats d and e in FIG. 2.

When constructing multispecific antibodies with scFvs assembled from VH and VL of the antibodies according to the present invention, cysteine (C) mutations may be introduced into the scFvs to improve the stability of the multispecific antibodies, for instance, cysteine mutations are introduced at position 44 of VH and position 100 of VL. As used herein, the term "cysteine (C) mutations at VH44 and VL100" refers to substitution of the amino acid at position 44 of VH of the scFv with cysteine and substitution of the amino acid at position 100 of VL of the scFv with cysteine.

The present invention also provides polynucleotide molecules encoding the aforementioned antibodies or fragments thereof. The polynucleotides of the present invention can be in DNA or RNA form. DNA forms include cDNA, genomic DNA, or synthetic DNA. DNA can be single-stranded or double-stranded. DNA can be the coding strand or the non-coding strand. The coding region sequence encoding the mature polypeptide can be the same as or a degenerate variant of the coding region sequence of the antibody of the present invention. As used herein, "degenerate variant" in the present invention refers to a nucleic acid sequence that encodes a polypeptide having the same amino acid sequence as the polypeptide of the present invention, but has a different coding region sequence.

Polynucleotides encoding the mature polypeptides of the present invention include: coding sequences encoding only the mature polypeptide; coding sequences for the mature polypeptide and various additional coding sequences; coding sequences for the mature polypeptide (and optionally additional coding sequences) and non-coding sequences.

The term "polynucleotide encoding a polypeptide" can be a polynucleotide that includes the coding sequence for this polypeptide, or it can be a polynucleotide that futher includes additional coding and/or non-coding sequences.

The present invention also relates to polynucleotides that hybridize to the above sequences and have at least 50%, preferably at least 70%, more preferably at least 80% identity between the two sequences. The present invention particularly relates to polynucleotides that can hybridize to the polynucleotides described in the present invention under stringent conditions. In the present invention, "stringent conditions" refers to: (1) hybridization and washing at low ionic strength and high temperature, such as 0.2×SSC, 0.1%SDS, 60°C; or (2) hybridization with a denaturant, such as 50% (v/v) formamide, 0.1% calf serum/0.1% Ficoll, 42°C, etc.; or (3) hybridization only occurs when the identity between the two sequences is at least 90%, preferably above 95%. Moreover, the polypeptides encoded by the polynucleotides that can hybridize have the same biological function and activity as the mature polypeptides shown in SEQ ID NO.4 and SEQ ID NO.9.

The full-length nucleotide sequence of the antibody of the present invention or a fragment thereof can generally be obtained by PCR amplification, recombination or artificial synthesis. A feasible method is to use artificial synthesis to synthesize the related sequences, especially when the fragment length is shorter. Usually, a very long fragment can be obtained by first synthesizing multiple small fragments and then ligating them. In addition, the coding sequence of the heavy chain can be fused with an expression tag (such as 6His) to form a fusion protein.

Once the relevant sequence is obtained, the relevant sequence can be obtained in large quantities by recombination. This is usually done by cloning it into a vector, transforming it into a cell, and then isolating the relevant sequence from the proliferated host cells by conventional methods. The biological molecules (nucleic acids, proteins, etc.) involved in the present invention include biological molecules that exist in an isolated form.

At present, it is possible to obtain the DNA sequence encoding the protein of the present invention (or its fragment, or its derivative) completely by chemical synthesis. The DNA sequence can then be introduced into various existing DNA molecules (or vectors) and cells known in the art. In addition, mutations can be introduced into the protein sequence of the present invention by chemical synthesis.

The present invention also relates to vectors comprising the appropriate DNA sequences described above and appropriate promoter or control sequences. These vectors can be used to transform appropriate host cells to enable them to express proteins.

The host cell can be a prokaryotic cell, such as a bacterial cell; or a lower eukaryotic cell, such as a yeast cell; or a higher eukaryotic cell, such as a mammalian cell. Representative examples include: *Escherichia coli*, *Streptomyces*; bacterial cells of *Salmonella typhimurium*; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as *Escherichia coli*, competent cells capable of taking up DNA can be harvested after the exponential growth phase and treated with the CaCl₂ method, the steps of which are well known in the art. Another method is to use MgCl₂. If necessary, transformation can also be carried out by electroporation. When the host is a eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the present invention. Depending on the host cell used, the culture medium used in the culture can be selected from various conventional culture media. The culture is carried out under conditions suitable for the growth of the host cell. When the host cells grow to an appropriate cell density, the selected promoter is induced by an appropriate method (such as temperature shift or chemical induction), and the cells are cultured for a further period of time.

In the above methods, the recombinant polypeptide can be expressed intracellularly, or on the cell membrane, or secreted extracellularly. If necessary, the recombinant protein can be separated and purified by various separation methods using its physical, chemical and other properties. These methods are well known to those skilled in the art. Examples of these methods include but are not limited to: conventional renaturation treatment, treatment with protein precipitants (salting out method), centrifugation, osmotic lysis, ultrafiltration, ultracentrifugation, molecular sieve chromatography (gel filtration), adsorption chromatography, ion exchange chromatography, high performance liquid chromatography (HPLC) and other various liquid chromatography techniques and combinations of these methods.

The antibodies of the present invention can be used alone or in combination or conjugated with detectable labels (for diagnostic purposes), therapeutic agents, PK (protein kinase) modification moieties, or any combination of the above substances.

Detectable labels for diagnostic purposes include but are not limited to: fluorescent or luminescent labels, radioactive labels, MRI (magnetic resonance imaging) or CT (computed tomography) contrast agents, or enzymes capable of producing detectable products.

Couplable therapeutic agents include but are not limited to: insulin, IL-2, interferon, calcitonin, GHRH peptide, enterostatin analogs, albumin, antibody fragments, cytokines, and hormones.

The present invention also provides a composition. In a preferred embodiment, the composition is a pharmaceutical composition, which comprises the aforementioned antibody or active fragment or fusion protein thereof, and a pharmaceutically acceptable carrier. Typically, these substances can be formulated in a non-toxic, inert and pharmaceutically acceptable aqueous carrier medium, the pH of which is usually about 5-8, preferably about 6-8, although the pH value may vary depending on the nature of the substance being formulated and the disease to be treated. The prepared pharmaceutical composition can be administered by conventional routes, including but not limited to: oral, respiratory, intra-tumoral, intraperitoneal, intravenous, or topical administration.

The pharmaceutical composition of the present invention can be used for the treatment of cancer/tumors, especially solid tumors, especially solid tumors with high LCRR15 expression.

The pharmaceutical composition of the present invention comprises a safe and effective amount (such as 0.001-99wt%, preferably 0.01-90wt%, more preferably 0.1-80wt%) of the aforementioned monoclonal antibody (or conjugate thereof) of the present invention and a pharmaceutically acceptable carrier or excipient. Such carriers include but are not limited to: saline, buffers, glucose, water, glycerol, ethanol, and a combination thereof. The pharmaceutical formulation should be compatible with the route of administration. The pharmaceutical composition of the present invention can be prepared in the form of an injection, for example, by conventional methods using a physiological saline solution or an aqueous solution containing glucose and other excipients. The pharmaceutical composition, such as an injection or solution, should be manufactured under aseptic conditions. The dosage of the active ingredient is a therapeutically effective amount, for example about 1 microgram/kg body weight to about 10 milligrams/kg body weight per day. In addition, the pharmaceutical composition of the present invention can also be used together with other therapeutic agents.

When using the pharmaceutical composition, a safe and effective amount of the immunoconjugate is administered to a mammal, wherein the safe and effective amount is usually at least about 10 micrograms/kg body weight and in most cases does not exceed about 8 milligrams/kg body weight, preferably the dose is about 10 micrograms/kg body weight to about 1 milligram/kg body weight. Of course, the specific dosage should also consider the route of administration, the patient's health status, etc., which are all within the scope of the skills of a skilled physician.

### The main advantages of the present invention include:

(1) Cancer-associated fibroblasts (CAFs) serve as vital components within the microenvironment of most solid tumors. These cells facilitate cancer progression by sustaining tumor cell proliferation, remodeling the tumor extracellular matrix, promoting angiogenesis, and modulating tumor-triggered inflammation. CAFs coexist within the tumor stroma together with other cell types including endothelial cells and immune cells that express molecules such as CD40. Given the specific expression of LRRC15 on CAFs, bispecific, trispecific or tetraspecific antibodies can be designed against this target to deliver immune-activating signals into the tumor stroma, thereby facilitating the conversion of cold tumors into hot tumors during the treatment of most solid tumors.
(2) Compared with conventional tumor-associated antigen (TAA) targets, LRRC15 is expressed in a variety of solid tumors as well as CAFs, which endows the bispecific, trispecific and tetraspecific antibodies containing the LRRC15 antigen binding domain according to the present invention with broader application scope.
(3) Compared with monospecific antibodies, the bispecific, trispecific and tetraspecific antibodies of the present invention exhibit superior antitumor efficacy both *in vitro* and *in vivo* with improved safety profiles.

The present invention is further explained below in conjunction with specific examples. It should be understood that these examples are only for illustration of the present invention and not for limiting the scope of the present invention. The experimental methods not specifically mentioned in the following examples are generally carried out under conventional conditions, such as those described in Sambrook et al., Molecular Cloning: A Laboratory Manual (New York: Cold Spring Harbor Laboratory Press, 1989), or according to the conditions recommended by the manufacturer. Unless otherwise specified, percentages and parts are percentages by weight and parts by weight.

### Sequences used to prepare the multi-specific T cell engager of the present invention

### Sequences of LRRC15 antibodies

| | HCDR1 | HCDR2 | HCDR3 |
|---|---|---|---|
| L15-06H/ L15-06C H | GFTFSSYYMS (SEQ ID NO: 33) | DISVSGSYTNYADSV KG (SEQ ID NO: 34) | IGSYRSFFSTDDGDSL DY (SEQ ID NO: 35) |
| L15-08H | GFTFSDYYMS (SEQ ID NO: 36) | DISVSSSYTNYADSV KG (SEQ ID NO: 37) | LGGYRSFFSTDDGDSL DY (SEQ ID NO: 38) |
| L15-03H | GGTFSSYTIS (SEQ ID NO: 39) | GIIPMFGTANYAQKF QG (SEQ ID NO: 40) | GVALLATDFDY (SEQ ID NO: 41) |
| L15-07H | GGTFSTYAIS (SEQ ID NO: 42) | GITPMFGTPNYAQKF QG (SEQ ID NO: 43) | GDWYDYHFDY (SEQ ID NO: 44) |

| | LCDR1 | LCDR2 | LCDR3 |
|---|---|---|---|
| L15-06L/ L15-06C L | RASQSISSWLA (SEQ ID NO: 45) | DASSLES (SEQ ID NO: 46) | QQYNSYSLT (SEQ ID NO: 47) |
| L15-08L | RASQSISSWLA (SEQ ID NO: 45) | DAFSLES (SEQ ID NO: 48) | QQYNSYSIT (SEQ ID NO: 49) |
| L15-03L | TGTSSDVGGYNY VS (SEQ ID NO: 50) | EVSNRPS (SEQ ID NO: 51) | QSWTHDNSVV (SEQ ID NO: 52) |
| L15-07L | TGTSSDVGGYNY VS (SEQ ID NO: 50) | EVSNRPS (SEQ ID NO: 51) | QSYANRDNFVV (SEQ ID NO: 53) |

| | |
|---|---|
| EVQLLESGGGLVQPGGSLRLSCAASGFTFSSYYMSWVRQA PGKGLEWVADISVSGSYTNYADSVKGRFTISRDNSKNTLY LQMNSLRAEDTAVYYCARIGSYRSFFSTDDGDSLDYWGQ GTLVTVSS | VH; L15-06H (SEQ ID NO: 1) |
| QVQLVESGGGLVKPGGSLRLSCAASGFTFSDYYMSWIRQA PGKGLEWVSDISVSSSYTNYADSVKGRFTISRDNAKNSLY LQMNSLRAEDTAVYYCARLGGYRSFFSTDDGDSLDYWGQ GTLVTVSS | VH; L15-08H (SEQ ID NO: 2) |
| QVQLVQSGAEVKKPGSSVKVSCKASGGTFSSYTISWVRQA PGQGLEWMGGIIPMFGTANYAQKFQGRVTITADESTSTAY MELSSLRSEDTAVYYCARGVALLATDFDYWGQGTLVTVS S | VH; L15-03H (SEQ ID NO: 3) |
| QVQLVQSGAEVKKPGSSVKVSCKASGGTFSTYAISWVRQ APGQGLEWMGGITPMFGTPNYAQKFQGRVTITADESTSTA YMELSSLRSEDTAVYYCARGDWYDYHFDYWGQGTLVTV SS | VH; L15-07H (SEQ ID NO: 4) |
| EVQLLESGGGLVQPGGSLRLSCAASGFTFSSYYMSWVRQA PGKCLEWVADISVSGSYTNYADSVKGRFTISRDNSKNTLY LQMNSLRAEDTAVYYCARIGSYRSFFSTDDGDSLDYWGQ GTLVTVSS | VH; L15-06CH (SEQ ID NO: 5) |
| DIQMTQSPSTLSASVGDRVTITCRASQSISSWLAWYQQKPG KAPKLLIYDASSLESGVPSRFSGSGSGTEFTLTISSLQPDDF ATYYCQQYNSYSLTFGGGTKVEIK | VL; L15-06L (SEQ ID NO: 6) |
| DIQLTQSPSTLSASVGDRVTITCRASQSISSWLAWYQQKPG KAPKLLIYDAFSLESGVPSRFSGSGSGTEFTLTISSLQPDDF ATYYCQQYNSYSITFGGGTKVEIK | VL; L15-08L (SEQ ID NO: 7) |
| QSALTQPASVSGSPGQSITISCTGTSSDVGGYNYVSWYQQ HPGKAPKLMIYEVSNRPSGVSNRFSGSKSGNTASLTISGLQ AEDEADYYCQSWTHDNSVVFGGGTKLTVL | VL; L15-03L (SEQ ID NO: 8) |
| QSALTQPASVSGSPGQSITISCTGTSSDVGGYNYVSWYQQ HPGKAPKLMIYEVSNRPSGVSNRFSGSKSGNTASLTISGLQ AEDEADYYCQSYANRDNFVVFGGGTKLTVL | VL; L15-07L (SEQ ID NO: 9) |
| DIQMTQSPSTLSASVGDRVTITCRASQSISSWLAWYQQKPG KAPKLLIYDASSLESGVPSRFSGSGSGTEFTLTISSLQPDDF ATYYCQQYNSYSLTFGCGTKVEIK | VL; L15-06CL (SEQ ID NO: 10) |

### Sequences of CD40 antibodies

| | |
|---|---|
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGYCTNGVCSYFDY WGQGTLVTVSS | VH; CD40 (SEQ ID NO: 11) |
| DIQMTQSPSSVSASVGDRVTITCRASQGIYSWLAWYQQKP GKAPNLLIYTASTLQSGVPSRFSGSGSGTDFTLTISSLQPED FATYYCQQANIFPLTFGGGTKVEIK | VL; CD40 (SEQ ID NO: 12) |
| QVQLVQSGAEVKKPGASVKVSCKASGYTFTGYYMHWVR QAPGQGLEWMGWINPDSGGTNYAHKFQGRVTMTRDTSIS TAYMELNRLRSDDTAVYYCARDQPLGYCTNGVCSYFDY WGQGTLVTVSS | VH1(Q62H); CD40 (SEQ ID NO: 13) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGLCTNGVCSYFDY WGQGTLVTVSS | VH2(Y104L); CD40 (SEQ ID NO: 14) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGACTNGVCSYFDY WGQGTLVTVSS | VH3(Y104A); CD40 (SEQ ID NO: 15) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGYCGSGVCSYFDY WGQGTLVTVSS | VH4(T106G/N1 07S); CD40 (SEQ ID NO: 16) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGYCTGGVCSYFDY WGQGTLVTVSS | VH5(N107G); CD40 (SEQ ID NO: 17) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGYCTNGDCSYFDY WGQGTLVTVSS | VH6(V109D); CD40 (SEQ ID NO: 18) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGYCTNGVCSAFDY WGQGTLVTVSS | VH7(Y112A); CD40 (SEQ ID NO: 19) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGECTNGVCSYFDY WGQGTLVTVSS | VH11(Y104E); CD40 (SEQ ID NO: 20) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGSCTNGVCSYFDY WGQGTLVTVSS | VH12(Y104S); CD40 (SEQ ID NO: 21) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGGCTNGVCSYFDY WGQGTLVTVSS | VH13(Y104G); CD40 (SEQ ID NO: 22) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPAGACTNGVCSYFDY WGQGTLVTVSS | VH14(Y104A/L 102A); CD40 (SEQ ID NO: 23) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGACTNGVCSAFDY WGQGTLVTVSS | VH15(Y104A/Y 112A); CD40 (SEQ ID NO: 114) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGGCTNGVCSAFDY WGQGTLVTVSS | VH16(Y104G/Y 112A); CD40 (SEQ ID NO: 115) |
| QVQLVQSGAEVKKPGASVKVSCKASGYTFTGYYMHWVR QAPGQGLEWMGWINPDSGGTNYAQKFQGRVTMTRDTSIS TAYMELNRLRSDDTAVYYCARDQPLGECTNGVCSAFDY WGQGTLVTVSS | VH17(Y104E/Y 112A); CD40 (SEQ ID NO: 116) |
| DIQMTQSPSSVSASVGDRVTITCRASQGIYSWLAWYQQKP GKAPNLLIYTASTLQSGVPSRFSGSGSGTDFTLTISSLQPED FATYYCQQASIFPLTFGGGTKVEIK | VL8(N92S); CD40 (SEQ ID NO: 24) |
| DIQMTQSPSSVSASVGDRVTITCRASQGIYSWLAWYQQKP GKAPNLLIYTASTLQSGVPSRFSGSGSGTDFTLTISSLQPED FATYYCQQANDFPLTFGGGTKVEIK | VL9(I93D); CD40 (SEQ ID NO: 25) |
| DIQMTQSPSSVSASVGDRVTITCRASQGIYSWLAWYQQKP GKAPNLLIYTASTLQSGVPSRFSGSGSGTDFTLTISSLQPED FATYYCQQANISPLTFGGGTKVEIK | VL10(F94S); CD40 (SEQ ID NO: 26) |
| | VH; CD40d (SEQ ID NO: 27) |
| | VL; CD40d (SEQ ID NO: 28) |

### Sequences of PD-1 antibody

| | |
|---|---|
| QVQLVQSGAEVKKPGASVKVSCKASGYTFTSYYIHWVRQ APGQGLEWMGWIFPGSGNSKYNENFKGRVTLTADTSTST VYMELSSLRSEDTAVYYCASETYDYGDYWGQGTLVTVSS | VH; P1 (SEQ ID NO: 29) |
| DIQMTQSPSFLSASVGDRVTITCKASQNVGTNVAWYQQK PGKAPKALIYSASYRYSGVPSRFSGSGSGTEFTLTISSLQPE DFATYYCQQYYSYPYTFGQGTKLEIK | VL; P1 (SEQ ID NO: 30) |

### Sequences of Claudin 18.2 antibody

| | |
|---|---|
| QVQLVQSGAEVKKPGASVKVSCKASGYTFTNSGMNWVR QAPGQGLEWMGWINTNTGEPTFAEEFRGRVTMTRDTSIST AYMELSRLRSDDTAVYYCARYYYGNSFAYWGQGTLVTV SS | VH;18.2 (SEQ ID NO: 31) |
| DIVMTQSPDSLAVSLGERATINCKSSQSLLNSGNQKNYLT WYQQKPGQPPKLLIYWASTRESGVPDRFSGSGSGTDFTLT ISSLQAEDVAVYYCQNNYFYPLTFGGGTKVEIK | VL;18.2 (SEQ ID NO: 32) |

### Sequences of CD3 antibodies

| | |
|---|---|
| EVQLVESGGGLVQPGGSLRLSCAASGFTFSTYAMNWVRQ APGKGLEWVGRIRSKYNNYATYYADSVKGRFTISRDDSK NTLYLQMNSLRAEDTAVYYCVRHGNFGDSYVSWFEYWG QGTLVTVSS | VH; CD3 (SEQ ID NO: 82) |
| EVQLVESGGGLVQPGGSLRLSCAASGFTFSTYAMNWVRQ APGKGLEWVGRIRSKANNYATYYADSVKGRFTISRDDSK NTLYLQMNSLRAEDTAVYYCVRHGNFGDSYVSWFEYWG QGTLVTVSS | VH; CD3v1 (SEQ ID NO: 83) |
| EVQLVESGGGLVQPGGSLRLSCAASGFTFSTYAMNWVRQ APGKGLEWVGRIRSKYNNYATYYADSVKGRFTISRDDSK NTLYLQMNSLRAEDTAVYYCVRHGNFGDSYVSWFDYWG QGTLVTVSS | VH; CD3v2 (SEQ ID NO: 84) |
| EVQLVESGGGLVQPGGSLRLSCAASGFTFSTYAMNWVRQ APGKGLEWVGRIRSEANNYATYYADSVKGRFTISRDDSK NTLYLQMNSLRAEDTAVYYCVRHGNFGDSYVSWFEYWG QGTLVTVSS | VH; CD3v3 (SEQ ID NO: 117) |
| QAVVTQEPSLTVSPGGTVTLTCGSSTGAVTTSNYANWVQ QKPGKSPRGLIGGTNKRAPGVPARFSGSLLGGKAALTISG AQPEDEADYYCALWYSNHWVFGGGTKLTVL | VL; CD3 (SEQ ID NO: 85) |

### Sequences of FAP antibodies

| | |
|---|---|
| EVQLLESGGGLVQPGGSLRLSCAASGFTFSSYAMSWVRQ APGKGLEWVSAIIGSGASTYYADSVKGRFTISRDNSKNTL YLQMNSLRAEDTAVYYCAKGWFGGFNYWGQGTLVTVSS | VH; F1 (SEQ ID NO: 86) |
| EIVLTQSPGTLSLSPGERATLSCRASQSVTSSYLAWYQQKP GQAPRLLINVGSRRATGIPDRFSGSGSGTDFTLTISRLEPED FAVYYCQQGIMLPPTFGQGTKVEIK | VL; F1 (SEQ ID NO: 87) |
| EVQLLESGGGLVQPGGSLRLSCAASGFTFSSHAMSWVRQ APGKGLEWVSAIWASGEQYYADSVKGRFTISRDNSKNTL YLQMNSLRAEDTAVYYCAKGWLGNFDYWGQGTLVTVSS | VH; F2 (SEQ ID NO: 88) |
| EIVLTQSPGTLSLSPGERATLSCRASQSVSRSYLAWYQQKP GQAPRLLIIGASTRATGIPDRFSGSGSGTDFTLTISRLEPEDF AVYYCQQGQVIPPTFGQGTKVEIK | VL; F2 (SEQ ID NO: 89) |
| QVQLVQSGAEVKKPGASVKVSCKASGYTFTNNGINWLRQ APGQGLEWMGEIYPRSTNTLYAQKFQGRVTITADRSSNTA YMELSSLRSEDTAVYFCARTLTAPFAFWGQGTLVTVSS | VH; F3 (SEQ ID NO: 90) |
| QIVLTQSPATLSLSPGERATLSCSASSGVNFMHWYQQKPG QAPKRLIFDTSKLASGVPARFSGSGSGTDYTLTISSLEPEDF AVYYCQQWSFNPPTFGQGTKVEIK | VL; F3 (SEQ ID NO: 91) |
| QVQLVQSGAEVKKPGASVKVSCKTSRYTFTEYTIHWVRQ APGQRLEWIGGINPNNGIPNYNQKFKGRVTITVDTSASTA YMELSSLRSEDTAVYYCARRRIAYGYDEGHAMDYWGQG TLVTVSS | VH; F4 (SEQ ID NO: 92) |
| DIVMTQSPDSLAVSLGERATINCKSSQSLLYSRNQKNYLA WYQQKPGQPPKLLIFWASTRESGVPDRFSGSGFGTDFTLTI SSLQAEDVAVYYCQQYFSYPLT FGQGTKVEIK | VL; F4 (SEQ ID NO: 93) |

### TGF-βRII sequences

| | |
|---|---|
| MIVPPNNGAVKFPQLCKFCDVRFSTCDNQKSCMSNCSITSI CEKPQEVCVAVWRKNDENITLETVCHDPKLPYHDFILEDA ASPKCIMKEKKKPGETFFMCSCSSDECNDNIIFSEEYNTSN PD | b1 (SEQ ID NO: 94) |
| GIPPHVQKSVNNDMIVTDNNGAVKFPQLCKFCDVRFSTCD NQKSCMSNCSITSICEKPQEVCVAVWRKNDENITLETVCH DPKLPYHDFILEDAASPKCIMKEKKKPGETFFMCSCSSDEC NDNIIFSEEYNTSNPD | b2 (SEQ ID NO: 95) |

### Sequence of IgG1 CH

| | |
|---|---|
| ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRVEPKSCDKTHTCPPCPAPELLG GPSVFLFPPKPKDTLMISRTPEVTCVVVDVSHEDPEVKF NWYVDGVEVHNAKTKPREEQYNSTYRVVSVLTVLHQD WLNGKEYKCKVSNKALPAPIEKTISKAKGQPREPQVYT LPPSRDELTKNQVSLTCLVKGFYPSDIAVEWESNGQPE NNYKTTPPVLDSDGSFFLYSKLTVDKSRWQQGNVFSCS VMHEALHNHYTQKSLSLSPGK | CH1-3 IgG1 (SEQ ID NO: 54) |

The **bold** part is the Fc region.

### CH1 sequences

| | |
|---|---|
| ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKRV | IgG1 CH1(SEQ ID NO: 55) |
| ASVAAPSVFIFPPSDEQLKSGTASVVCLLNNFYPREAKVQ WKVDNALQSGNSQESVTEQDSKDSTYSLSSTLTLSKADYE KHKVYACEVTHQGLSSPVTKSFNRGE | IgG1 CH1-CL Cross (SEQ ID NO: 56) |

### Fc region sequences

| | |
|---|---|
| EPKSCDKTHTCPPCPAPELLGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALPAPIEK TISKAKGQPREPQVYTLPPSRDELTKNQVSLTCLVKGFYPS DIAVEWESNGQPENNYKTTPPVLDSDGSFFLYSKLTVDKS RWQQGNVFSCSVMHEALHNHYTQKSLSLSPGK | IgG1 Fc (SEQ ID NO: 57) |
| CDKTHTCPPCPAPELLGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALPAPIEKTISK AKGQPREPQVYTLPPSRDELTKNQVSLTCLVKGFYPSDIA VEWESNGQPENNYKTTPPVLDSDGSFFLYSKLTVDKSRW QQGNVFSCSVMHEALHNHYTQKSLSLSPGK | IgG1 Fc (SEQ ID NO: 58) |

The Fc region can contain the following mutations:

| Mutations to knock out FcγR function and/or the KIH or charged pairs mutations | Mutations to increase the Fcγ RIIb binidng |
|---|---|
| N297A, L234F/L235E/P331S, L234A/L235A/P329G | S267E, S267E/G236D, S267E/S239D, S267E/L328F |
| Y349C/K370E/K409D/K439E, S354C/D356K/E357K/D399K | |
| S354C/T366W, Y349C/T366S/L368A/Y407V, L234F/L235E/P331S/Y349C/K370E/K409D/K439E | |
| L234F/L235E/P331S/S354C/D356K/E357K/D399K | |
| L234F/L235E/P331S/S354C/T366W | |
| L234F/L235E/P331S/Y349C/T366S/L368A/Y407V | |
| L234A/L235A/P329G/Y349C/K370E/K409D/K439E | |
| L234A/L235A/P329G/S354C/D356K/E357K/D399K | |
| L234A/L235A/P329G/S354C/T366W | |
| L234A/L235A/P329G/Y349C/T366S/L368A/Y407V | |
| L234F/L235E/P331S/S354C/T366W | |
| L234F/L235E/P331S/Y349C/T366S/L368A/Y407V | |
| N297A/Y349C/K370E/K409D/K439E | |
| N297A/S354C/D356K/E357K/D399K | |
| N297A/S354C/T366W | |
| N297A/Y349C/T366S/L368A/Y407V | |

In a preferred embodiment of the present invention, the Fc region has the following sequences:

| | |
|---|---|
| EPKSCDKTHTCPPCPAPEAAGGPSVFLFPPKPKDTLMISRT PEVTCVVVDVSHEDPEVKFNWYVDGVEVHNAKTKPREE QYNSTYRVVSVLTVLHQDWLNGKEYKCKVSNKALGAPIE KTISKAKGQPREPQVYTLPPSRDELTKNQVSLTCLVKGFY PSDIAVEWESNGQPENNYKTTPPVLDSDGSFFLYSKLTVD KSRWQQGNVFSCSVMHEALHNHYTQKSLSLSPGK | Fc(L234A/L235 A/P329G) (SEQ ID NO: 59) |
| EPKSCDKTHTCPPCPAPEAAGGPSVFLFPPKPKDTLMISRT PEVTCVVVDVSHEDPEVKFNWYVDGVEVHNAKTKPREE QYNSTYRVVSVLTVLHQDWLNGKEYKCKVSNKALGAPIE KTISKAKGQPREPQVYTLPPCRKKLTKNQVSLTCLVKGFY PSDIAVEWESNGQPENNYKTTPPVLKSDGSFFLYSKLTVD KSRWQQGNVFSCSVMHEALHNHYTQKSLSLSPGK | Fc(L234A/L235 A/P329G/S354C /D356K/E357K/ D399K) (SEQ ID NO: 60) |
| EPKSCDKTHTCPPCPAPEAAGGPSVFLFPPKPKDTLMISRT PEVTCVVVDVSHEDPEVKFNWYVDGVEVHNAKTKPREE QYNSTYRVVSVLTVLHQDWLNGKEYKCKVSNKALGAPIE KTISKAKGQPREPQVCTLPPSRDELTKNQVSLTCLVEGFYP SDIAVEWESNGQPENNYKTTPPVLDSDGSFFLYSDLTVDK SRWQQGNVFSCSVMHEALHNHYTQESLSLSPGK | Fc(L234F/L235E /S267E/P331S) (SEQ ID NO: 61) |
| CDKTHTCPPCPAPEAAGGPSVFLFPPKPKDTLMISRTPEVT CVVVDVSHEDPEVKFNWYVDGVEVHNAKTKPREEQYNS TYRVVSVLTVLHQDWLNGKEYKCKVSNKALGAPIEKTIS KAKGQPREPQVCTLPPSRDELTKNQVSLTCLVEGFYPSDIA VEWESNGQPENNYKTTPPVLDSDGSFFLYSDLTVDKSRW QQGNVFSCSVMHEALHNHYTQESLSLSPGK | Fc(L234A/L235 A/P329G/Y349C /K370E/K409D/ K439E) (SEQ ID NO: 62) |
| EPKSCDKTHTCPPCPAPEAAGGPSVFLFPPKPKDTLMISRT PEVTCVVVDVSHEDPEVKFNWYVDGVEVHNAKTKPREE QYNSTYRVVSVLTVLHQDWLNGKEYKCKVSNKALGAPIE KTISKAKGQPREPQVCTLPPSRDELTKNQVSLTCLVEGFYP SDIAVEWESNGQPENNYKTTPPVLDSDGSFFLYSDLTVDK SRWQQGNVFSCSVMHEALHNHYTQESLSLSPGK | Fc(L234A/L235 A/P329G/Y349C /K370E/K409D/ K439E) (SEQ ID NO: 96) |
| EPKSSDKTHTCPPCPAPEAAGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALGAPIEK TISKAKGQPREPQVYTLPPCRKKLTKNQVSLTCLVKGFYP SDIAVEWESNGQPENNYKTTPPVLKSDGSFFLYSKLTVDK SRWQQGNVFSCSVMHEALHNHYTQKSLSLSPGK | Fc(C220S/L234 A/L235A/P329G /S354C/D356K/ E357K/D399K) (SEQ ID NO: 97) |
| EPKSSDKTHTCPPCPAPEAAGGPSVFLFPPKPKDTLMISRTP EVTCVVVDVSHEDPEVKFNWYVDGVEVHNAKTKPREEQ YNSTYRVVSVLTVLHQDWLNGKEYKCKVSNKALGAPIEK TISKAKGQPREPQVCTLPPSRDELTKNQVSLTCLVEGFYPS DIAVEWESNGQPENNYKTTPPVLDSDGSFFLYSDLTVDKS RWQQGNVFSCSVMHEALHNHYTQESLSLSPGK | Fc(C220S/L234 A/L235A/P329G /Y349C/K370E/ K409D/K439E) (SEQ ID NO: 98) |

### Sequence of IgG4 CH

| | |
|---|---|
| ASTKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVF LFPPKPKDTLMISRTPEVTCVVVDVSQEDPEVQFNWYV DGVEVHNAKTKPREEQFNSTYRVVSVLTVLHQDWLNG KEYKCKVSNKGLPSSIEKTISKAKGQPREPQVYTLPPSQ KKMTKNQVSLTCLVKGFYPSDIAVEWESNGQPENNYK TTPPVLKSDGSFFLYSRLTVDKSRWQEGNVFSCSVMHE ALHNHYTQKSLSLSLGK | CH1-3 IgG4 (S228P) (SEQ ID NO: 63) |
| | CH1 : SEQ ID NO: 64 |
| | Fc:SEQ ID NO: 65 |

The **bold** part is the Fc region.

The Fc region can contain the following mutations:

| Mutations of the KIH or the charged pairs | Mutations to increase the Fcγ RIIb binidng |
|---|---|
| Y349C/K370E/R409D/K439E, S354C/E356K/E357K/D399K, S354C/T366W, Y349C/T366S/L368A/Y407V | S228P/S267E, S228P/S267E/G236D, S228P/S267E/S239D, S228P/S267E/L328F |

In another preferred embodiment of the present invention, the C-terminal lysine (K) of the aforementioned Fc region is deleted to reduce the formation of antibody charge variants.

### Sequences of CL

| | |
|---|---|
| | CL; Kappa (SEQ ID NO: 66) |
| GQPKAAPSVTLFPPSSEELQANKATLVCLISDFYPGAVTVA WKADSSPVKAGVETTTPSKQSNNKYAASSYLSLTPEQWK SHRSYSCQVTHEGSTVEKTVAPTECS | CL; Lambda (SEQ ID NO: 67) |
| ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKVEPKSC | CL-CH1 Cross (SEQ ID NO: 68) |

### Optional linker sequences

| |
|---|
| G |
| GS |
| GSS |
| GSSSG |
| GGGGS |
| GGGGSGGGGS |
| GGGGSGGGGSGGGGS |
| GGGGSGGGGSGGGGSGGGGS |
| GGGGSGGGGSGGGGSGGGGSGGGGS |
| PGGGGSP |
| PGGGGSPGGGGSPGGGGSP |
| GEPGSGE |
| GEPGSGEGEPGSGE |
| GEPGSGEGEPGSGEEGEPGSGE |
| EGEPGSGEEGEPGSGEEGEPGSGEEGEPGSGE |
| GKPGS |
| GKPGSGKPGS |
| GKPGSGKPGSGKPGS |
| GKPGSGKPGSGKPGSGKPGS |
| GKPGSGKPGSGKPGSGKPGSGKPGS |
| SSSSG |
| SSSSGSSSSG |
| SSSSGSSSSGSSSSG |
| SSSSGSSSSGSSSSGSSSSG |
| GRPGSGPGSGRPGSGRPGS |
| GRPGSGPGSGRPGSGRPGSGRGPS |
| GKPGSGRPGSGKGPSGRPGS |

### Example 1 Preparation of the multispecific antibodies of the present invention

A variety of multispecific antibody molecules were designed based on four LRRC15 antibody sequences (L15-06, L15-08, L15-03 and L15-07) obtained by phage screening, mutant sequences of L15-06 (L15-06C), CD40 antibody sequences, PD-1 antibody sequences, Claudin18.2 antibody sequences, CD3 antibody sequences, FAP antibody sequences and TGF-βRII sequences.

Gene fragments encoding the sequences of the multispecific antibodies were obtained by full-gene synthesis. The target sequences were subsequently inserted into expression vectors via standard gene cloning techniques (see, e.g., Lo. B.K.C, Methods in Molecular Biology, Volume 248, 2004, Antibody Engineering).

HEK293E cells were transfected with the vectors carrying the genes encoding the multispecific molecules. The cells were cultured for 7 days at 37 °C under 5% CO₂ in F17 medium (1 L F17 medium + 10 mL 10% PF68 + 30 mL 200 mM L-glutamine) to produce the corresponding target molecules. After the completion of expression, cell culture supernatants were harvested and purified to obtain multispecific T cell engager molecules for subsequent experimental assays.

The following exemplary multispecific molecules were prepared using the above procedures:
Bispecific antibodies composed of an LRRC15 antigen binding domain and a CD40 antigen binding domain with structures shown as panels a and b in FIG. 1; trispecific antibodies composed of an LRRC15 antigen binding domain, a CD40 antigen binding domain and a PD-1 or Claudin18.2 antigen binding domain with structures shown as panels c and d in FIG. 1.

One bispecific antibody adopting format a in FIG. 1 is designated 1540s, with its structure illustrated in FIG. 1A; another bispecific antibody adopting format a is designated 1540s-3, with its structure illustrated in FIG. 1B. Two additional bispecific antibodies of format a are designated 1540s-7 and 1540s-16.

One bispecific antibody adopting format b in FIG. 1 is designated 40d15, with its structure illustrated in FIG. 1C.

One trispecific antibody adopting format c in FIG. 1 is designated 151840-L06, with its structure illustrated in FIG. 1D; another trispecific antibody adopting format c is designated 15P140-L06, with its structure illustrated in FIG. 1E.

One trispecific antibody adopting format d in FIG. 1 is designated 40d1815, with its structure illustrated in FIG. 1F; another trispecific antibody adopting format d is designated 40dP115, with its structure illustrated in FIG. 1G.

Bispecific antibodies composed of an LRRC15 antigen binding domain and a CD3 antigen binding domain with the structure shown as panel a in FIG. 2; trispecific antibodies composed of an LRRC15 antigen binding domain, a CD3 antigen binding domain and an FAP antigen binding domain with structures shown as panels b, c, f, g, h, i, j and k in FIG. 2; tetraspecific antibodies composed of an LRRC15 antigen binding domain, a CD3 antigen binding domain, an FAP antigen binding domain and a TGF-β binding domain with structures shown as panels d and e in FIG. 2.

One bispecific antibody adopting format a in FIG. 2 is designated 153, with its structure illustrated in FIG. 2A.

One trispecific antibody adopting format b in FIG. 2 is designated 315F, with its structure illustrated in FIG. 2B.

One trispecific antibody adopting format c in FIG. 2 is designated F315, with its structure illustrated in FIG. 2C.

One tetraspecific antibody adopting format d in FIG. 2 is designated 315Fb, with its structure illustrated in FIG. 2D.

One tetraspecific antibody adopting format e in FIG. 2 is designated F315b, with its structure illustrated in FIG. 2E.

One trispecific antibody adopting format f in FIG. 2 is designated F153, with its structure illustrated in FIG. 2F.

One trispecific antibody adopting format g in FIG. 2 is designated 15F3, with its structure illustrated in FIG. 2G.

One trispecific antibody adopting format h in FIG. 2 is designated 153F, with its structure illustrated in FIG. 2H.

One trispecific antibody adopting format i in FIG. 2 is designated 1533F, with its structure illustrated in FIG. 2I.

One trispecific antibody adopting format j in FIG. 2 is designated F3153, with its structure illustrated in FIG. 2J.

One trispecific antibody adopting format k in FIG. 2 is designated F3315, with its structure illustrated in FIG. 2K.

Exemplary molecules prepared in this Example are listed as follows:

| Molecule Name | Chain 1 (SEQ ID NO:) | Chain 2 (SEQ ID NO:) | Chain 3 (SEQ ID NO:) | Chain 4 (SEQ ID NO:) |
|---|---|---|---|---|
| 1540s | 69 | 70 | | |
| 1540s-3 | 71 | 70 | | |
| 1540s-7 | 118 | 70 | | |
| 1540s-16 | 119 | 70 | | |
| 40d15 | 72 | 73 | | |
| 151840-L06 | 74 | 70 | 75 | 76 |
| 15P140-L06 | 74 | 70 | 77 | 78 |
| 40d1815 | 79 | 73 | 80 | |
| 40dP115 | 79 | 73 | 81 | |
| 153 | 99 | 98 | | |
| 315F | 99 | 100 | 101 | |
| F315 | 102 | 103 | 70 | |
| 315Fb | 104 | 105 | 101 | |
| F315b | 106 | 107 | 70 | |
| F153 | 108 | 98 | | |
| 15F3 | 109 | 98 | | |
| 153F | 99 | 110 | | |
| 1533F | 99 | 111 | | |
| F3153 | 102 | 112 | | |
| F3315 | 102 | 113 | | |

### Example 2 Determination of binding affinity between bispecific antibodies targeting LRRC15 and CD40 and CD40

The binding affinities of bispecific antibody 1540s and its mutants 1540s-1 to 1540s-17 toward CD40 were determined. Mutants 1540s-1 to 1540s-17 share the identical structure with 1540s, with the only difference that variable region sequences within the CD40 antigen binding domain are substituted with VH1-7, VL8-10 and VH11-17 respectively.

Test antibodies were diluted with Octet Buffer (1×PBS + 0.02% Tween), and dispensed into a black 96-well plate at a volume of 200 µL/well. Serially diluted hCD40-His protein prepared with Octet Buffer was added into the aforementioned black 96-well plate at 200 µL/well. Octet Buffer was added to the plate at 200 µL/well, followed by regeneration solution (glycine solution) loaded at 200 µL/well. A sensor tray was prepared, each designated sensor well was filled with 200 µL Octet Buffer, and AHC2.0 assay probes were placed into corresponding sensor wells. The black 96-well plate together with the sensor tray was loaded into the ForteBio instrument for detection. Data were analyzed via OctetBLIAnalysis software to calculate KD values. The results are presented in Table 1-1 to Table 1-3 below.

The results demonstrated that all tested 1540s mutants exhibited reduced binding affinity for CD40 compared with the parental molecule. The binding signals of certain mutants to CD40 were too weak to acquire reliable quantitative measurements, which were marked as NA.

**Table 1-1**

| Molecule | KD (M) | kon (1/Ms) | kdis (1/s) |
|---|---|---|---|
| Seli | 6.32E-10 | 1.56E+05 | 9.84E-05 |
| 1540s | 5.67E-09 | 7.42E+04 | 4.21E-04 |
| 1540s-2 | 5.70E-09 | 9.98E+04 | 5.69E-04 |
| 1540s-6 | NA | | |
| 1540s-7 | 3.50E-08 | 1.09E+05 | 3.81E-03 |
| 1540s-8 | 6.64E-09 | 1.01E+05 | 6.68E-04 |
| 1540s-9 | NA | | |

**Table 1-2**

| Molecule | KD (M) | kon (1/Ms) | kdis (1/s) |
|---|---|---|---|
| Seli | 8.08E-10 | 1.20E+05 | 9.73E-05 |
| 1540s | 9.53E-09 | 6.76E+04 | 6.45E-04 |
| 1540s-1 | 1.07E-08 | 6.82E+04 | 7.31E-04 |
| 1540s-3 | 1.57E-08 | 8.01E+04 | 1.26E-03 |
| 1540s-4 | NA | | |
| 1540s-5 | NA | | |
| 1540s-10 | NA | | |

**Table 1-3**

| Molecule | KD (M) | kon (1/Ms) | kdis (1/s) |
|---|---|---|---|
| Seli | 1.99E-09 | 9.06E+04 | 1.80E-04 |
| 1540s | 1.40E-08 | 5.56E+04 | 7.80E-04 |
| 1540s-3 | 2.20E-08 | 6.55E+04 | 1.44E-03 |
| 1540s-11 | 4.45E-08 | 5.06E+04 | 2.25E-03 |
| 1540s-12 | 3.31E-08 | 6.20E+04 | 2.05E-03 |
| 1540s-13 | 4.44E-08 | 8.42E+04 | 3.73E-03 |
| 1540s-14 | NA | | |

**Table 1-4**

| Molecule | KD (M) | kon (1/Ms) | kdis (1/s) |
|---|---|---|---|
| Seli | 6.70E-10 | 8.45E+04 | 5.66E-05 |
| 1540s-3 | 1.25E-08 | 5.57E+04 | 6.95E-04 |
| 1540s-7 | 8.17E-08 | 3.64E+04 | 2.97E-03 |
| 1540s-15 | 2.66E-07 | 2.40E+04 | 6.38E-03 |
| 1540s-16 | 2.38E-07 | 2.81E+04 | 6.67E-03 |
| 1540s-17 | 3.67E-07 | 2.27E+04 | 8.33E-03 |

### Example 3 Functional Assays of Bispecific Antibodies Targeting LRRC15 and CD40

### 3.1 Activation of dendritic cells (DCs) by bispecific antibodies

Monocytes were isolated from PBMCs using a monocyte isolation kit (cat#130-096-537, Miltenyi Biotec). Monocytes were induced to differentiate into DCs upon treatment with 20 ng/mL GM-CSF and IL-4, and DCs were harvested after 7 days of culture. CHO cells overexpressing LRRC15 (wild-type CHO cells without LRRC15 expression served as control cells) were washed with PBMC culture buffer (RPMI-1640 + 10% FBS + 1% Penstrep), then resuspended at a density of 2×10⁶ cells/mL together with serially diluted test antibodies. Prepared DCs were added to the cell suspension to achieve an effector-to-target (E/T) cell ratio of 3:1. The mixed cell suspension was incubated at 37 °C for 24 h. Culture supernatants were collected, and the concentration of IL-12p40 was quantified using an IL-12p40 detection kit.

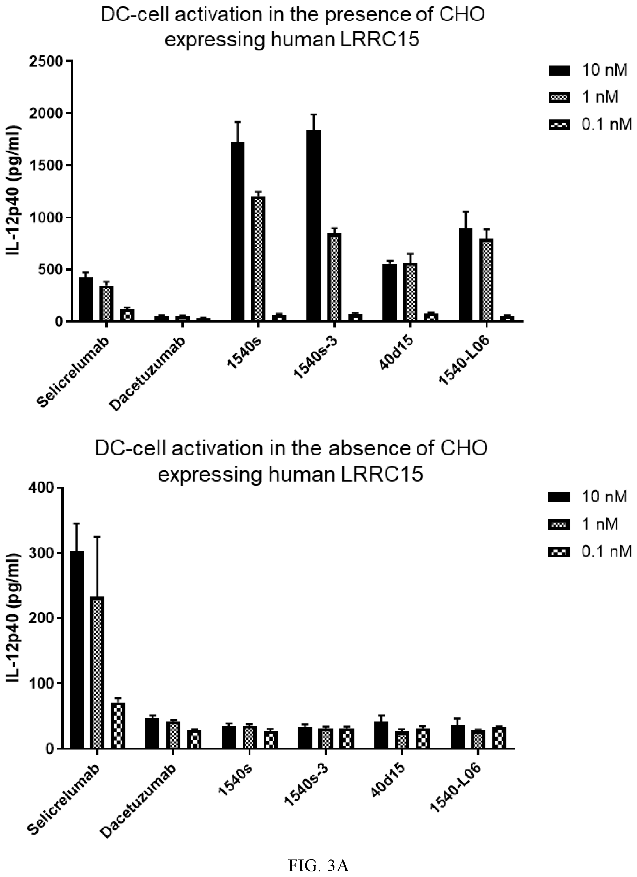

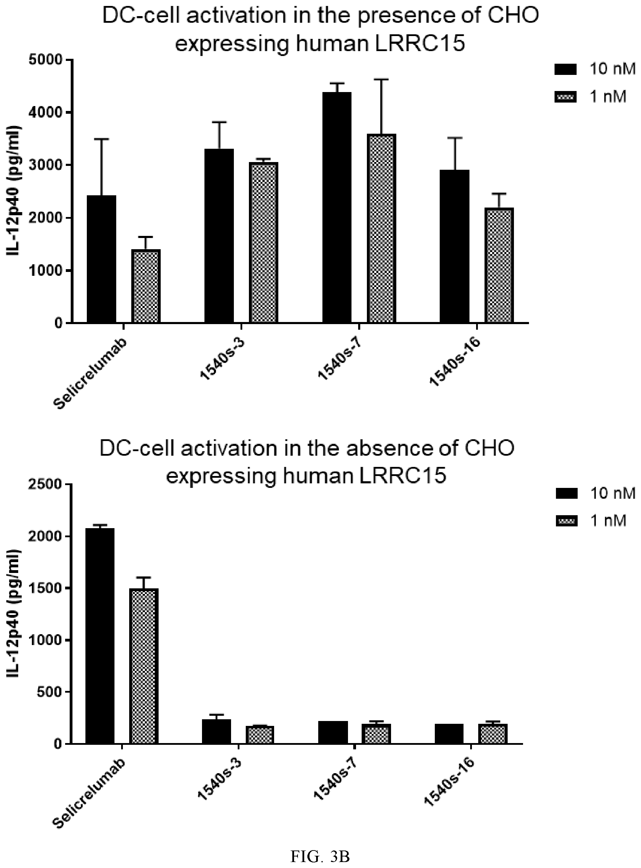

The test results are shown in FIG. 3A and FIG. 3B. The data in FIG. 3A revealed that all four bispecific antibodies triggered stronger DC activation compared with the control monoclonal antibodies. Among these candidates, 1540s and 1540s-3 exhibited superior activity relative to 1540-L06 (carrying Fc mutations L234F/L235E/P331S/S267E with identical amino acid sequences to 1540s in other regions; the full sequence is available in WO2023104214A1) and 40d15. None of the four bispecific antibodies induced obvious DC activation in the absence of target cells, demonstrating favorable safety performance. FIG. 3B shows the detection results of 1540s-3, 1540s-7, 1540s-16 and control monoclonal antibody Selicrelumab using DCs differentiated from PBMCs derived from another independent donor. Consistently, the three bispecific antibodies induced markedly higher activation signals than the monoclonal antibody control. No obvious non-specific DC activation was observed for the three bispecific antibodies without target cells, which represented improved safety compared with the control monoclonal antibody.

### 3.2.1 Activation of CD40 reporter gene by bispecific antibody molecules

CD40 reporter effector cells (Promega) in logarithmic growth phase were collected and centrifuged at 300 g for 5 min. The cell pellet was resuspended in 1 mL assay buffer (RPMI-1640 medium + 1% FBS), followed by cell counting; the cell density was adjusted to 1.5E5/ml with assay buffer.

After thorough mixing, the CD40 reporter cell suspension was seeded into a white 96-well plate at 100 µL/well, and incubated overnight (18-22 h) at 37 °C under 5% CO₂.

On the next day, CHO-hLRRC15 cells in logarithmic growth phase were harvested and centrifuged at 300 g for 5 min. Cells were resuspended in 1 mL assay buffer, counted, and diluted to 8E5/mL using assay buffer. The culture medium was gently aspirated from the 96-well plate pre-seeded with CD40 reporter cells on the previous day, and CHO-hLRRC15 cell suspension was added at 50 µL/well. Serially diluted test antibodies were dispensed into the plate at 25 µL/well with duplicate wells set up, and the plate was incubated in a CO₂ incubator for 5-6 h. BRITELITE PLUS reagent was added at 75 µL/well, the plate was incubated at room temperature in darkness for 5 min, and luminescence signals were measured with a microplate reader. GraphPad Prism software was applied for plotting and EC₅₀ calculation.

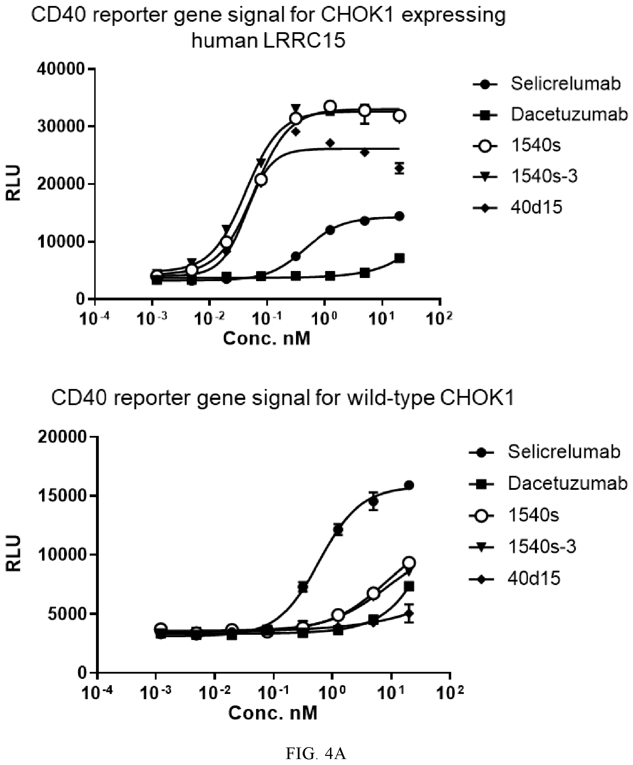

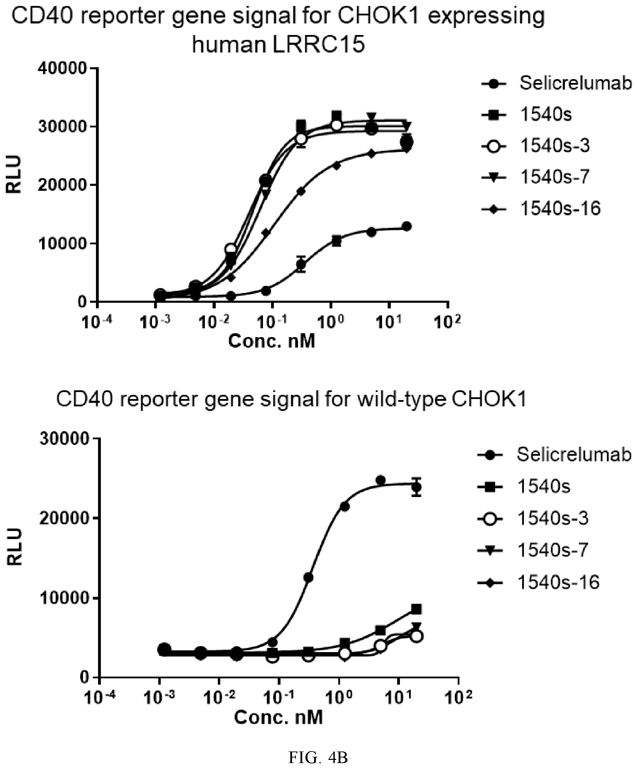

The results are shown in FIG. 4A and FIG. 4B. The data in FIG. 4A demonstrated that 1540s and 1540s-3 triggered robust activation of CD40 reporter signals in the presence of target cells, whereas their activation capacity decreased markedly when wild-type CHOK1 cells (without target antigen expression) were used. Among all candidates, 40d15 exhibited the weakest off-target activation without target cells, showing favorable safety profile together with superior efficacy relative to control monoclonal antibodies Selicrelumab and Dacetuzumab (the control antibodies are biosimilars having identical amino acid sequences to the reference antibodies of the same names). FIG. 4B displays results from an independent replicate assay comparing another panel of bispecific antibodies and control monoclonal antibodies. The candidates 1540s, 1540s-3, 1540s-7 and 1540s-16 elicited stronger reporter activation than the control monoclonal antibodies. They only induced marginal non-specific activation signals in the presence of wild-type CHOK1 cells compared with the control monoclonal antibodies, verifying satisfactory efficacy and safety performance.

### 3.2.2 Activation of CD3 reporter gene by trispecific antibody molecules

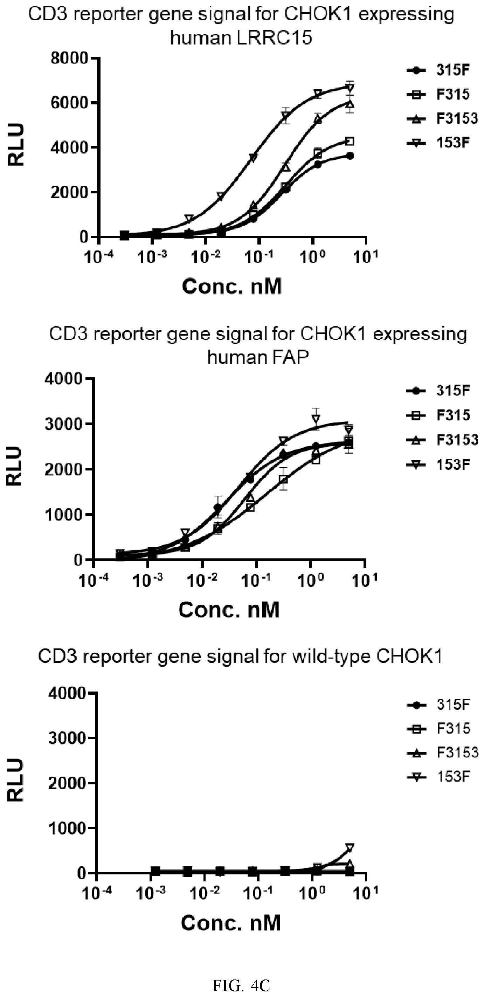

CD3 reporter effector cells (Promega) in logarithmic growth phase were adopted for this assay. The remaining experimental procedures were performed in accordance with the abovementioned CD40 reporter gene assay system. The results are shown in FIG. 4C. All four trispecific antibodies effectively activated the CD3 reporter gene system with low background signals in the absence of target cells (wild-type CHOK1 cells). In the presence of target cells expressing LRRC15 or FAP, 153F exhibited superior activation efficacy compared with other test molecules.

### 3.3 Capability of bispecific antibodies to induce cytokine secretion from PBMCs

The capacity of test antibodies to stimulate cytokine release from PBMCs was evaluated. Monoclonal antibody L15-06 (abbreviated as L06), Selicrelumab and Dacetuzumab were set as control samples, and anti-CD3 antibody OKT3 served as the positive control. The detailed experimental procedures are described as follows:
Cryopreserved PBMCs derived from three distinct donors (D1, D2, D3) were thawed, diluted to 10E7 cells/mL with PBMC culture buffer (RPMI-1640 + 10% FBS + 1% Penstrep) in a 96-well plate, and incubated at 37 °C for 48 h. Test antibodies were diluted to 10 nM using PBMC culture buffer and added into the 96-well plate containing PBMCs, followed by incubation at 37 °C for 24 h. The concentrations of cytokines in culture supernatants were quantified with the BD^{™} Cytometric Bead Array (CBA) kit to characterize the cytokine secretion profiles triggered by each antibody in PBMCs from different donors.

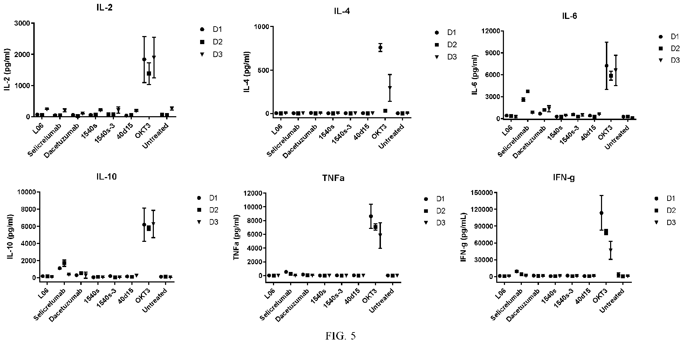

The results are shown in FIG. 5. The three bispecific antibodies did not induce obvious elevated secretion of the six detected cytokines, whereas the positive control anti-CD3 antibody OKT3 triggered varying degrees of cytokine release. The untreated group without antibody administration was used as the negative control and displayed negligible cytokine secretion.

### Example 4 In vivo efficacy evaluation of bispecific antibodies targeting LRRC15 and CD40

### 4.1 Efficacy in the KPC tumor model

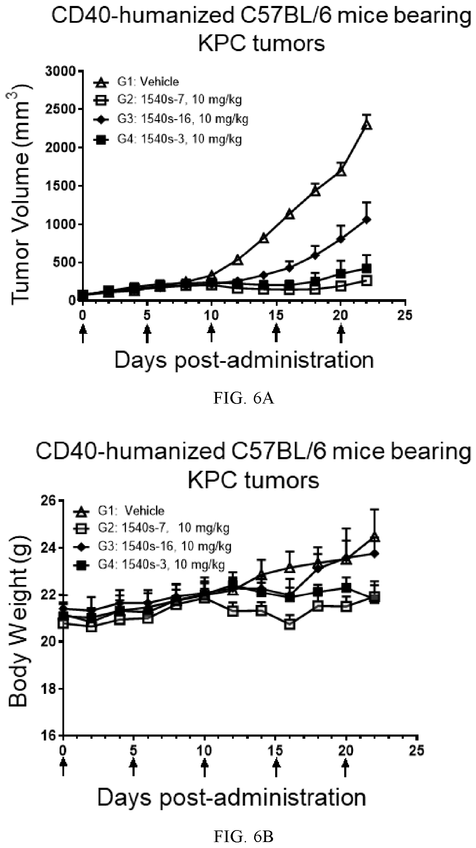

CD40 humanized C57BL/6 mice were inoculated with KPC cells at a dose of 5×10⁵ cells per mouse. When the average tumor volume reached approximately 170 mm³, the mice were randomized into different groups for administration (The control group received PBS, while the experimental groups were treated with 1540s-3, 1540s-7 and 1540s-16 respectively). The antibodies were administered intraperitoneally once every 5 days for a total of 5 doses at a dosage of 10 mg/kg. 1540s-3, 1540s-7 and 1540s-16 exerted significant antitumor effects with tumor growth inhibition (TGI) values of 82%, 88% and 54%, respectively (FIG. 6A). No remarkable body weight loss was observed in mice receiving any of the three bispecific antibodies (FIG. 6B), which verified favorable efficacy and safety profiles of these bispecific antibodies.

### 4.2 Efficacy in the MC38 tumor model

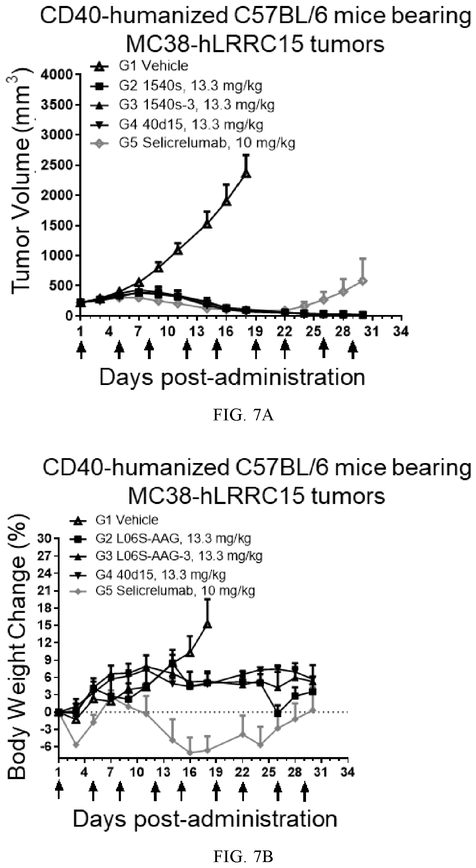

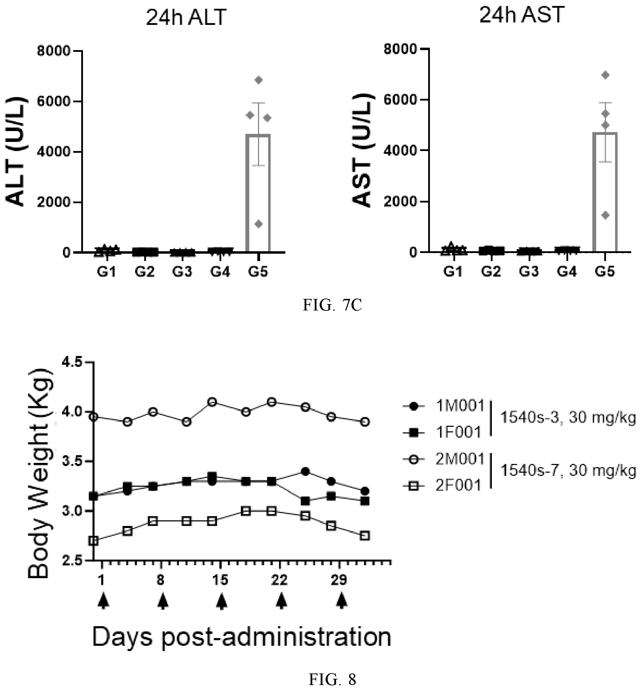

CD40 humanized C57BL/6 mice were inoculated with MC38-huLRRC15 cells at a dose of 1×10⁶ cells per mouse. Grouping and administration were initiated when the average tumor volume grew to around 220 mm³. The control group was treated with PBS, and the experimental groups received 1540s, 1540s-3 and 40d15 in accordance with the administration schedule illustrated in FIG. 7A. All three bispecific antibodies exhibited potent antitumor activity comparable to that of the control monoclonal antibody Selicrelumab (abbreviated as Seli). On Day 18 post administration, the TGI values were 106% for 1540s, 105% for 1540s-3, 107% for 40d15 and 106% for Selicrelumab (FIG. 7A). Long-term observation demonstrated sustained tumor growth suppression in the bispecific antibody treatment groups, whereas tumor relapse occurred in the Selicrelumab control group. In terms of body weight, the Selicrelumab group exhibited substantial body weight reduction. In contrast, 1540s, 1540s-3 and 40d15 induced no obvious body weight loss in mice, confirming superior safety of these bispecific antibodies relative to Selicrelumab (FIG. 7B). At 24 hours after administration, serum ALT and AST levels surged sharply in mice treated with Selicrelumab, indicating potential hepatotoxicity risks. By contrast, serum ALT and AST levels in the three bispecific antibody groups were comparable to those of the vehicle (PBS) group without evident elevation of ALT or AST (FIG. 7C).

### Example 5 Exploratory toxicology study in cynomolgus monkeys

Preliminary toxicological evaluations of 1540s-3 and 1540s-7 were performed in cynomolgus monkeys. The test articles were administered intravenously once weekly for 4 consecutive weeks (5 administrations in total) at a dose level of 30 mg/kg.

No obvious changes in body weight were observed throughout the study period (FIG. 8). No abnormal clinical signs were noted during routine clinical observation, and no gross macroscopic lesions were identified upon terminal necropsy. Serum cytokine levels were tested at 6 h, 24 h, 72 h and 168 h after the first administration, as well as at 24 h following the second to fifth administrations; no remarkable shifts in cytokine concentrations were detected at any time point. The IL-12 concentrations at all sampling time points remained below the lower limit of quantitation (LLOQ = 9.77 pg/mL), as summarized in Table 2. In contrast, published literature has reported that subcutaneous administration of Selicrelumab at 5 mg/kg markedly induces robust IL-12 secretion and leads to obvious splenomegaly in animals. Collectively, these data demonstrate that 1540s-3 and 1540s-7 exhibit substantially improved safety profiles compared with Selicrelumab.

**Table 2**

| Cytokines | | | | | |
|---|---|---|---|---|---|
| Index | Sampling Time Point | 1M001 | 1F001 | 2M001 | 2F001 |
| IL-2 (pg/mL) | | | | | |
| | Pre-first administration | BLQ | BLQ | BLQ | BLQ |
| | 6 h post first administration | BLQ | BLQ | BLQ | BLQ |
| | 24 h post first administration | BLQ | BLQ | BLQ | BLQ |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| IL-6 (pg/mL) | | | | | |
|---|---|---|---|---|---|
| | Pre-first administration | BLQ | BLQ | BLQ | BLQ |
| | 6 h post first administration | BLQ | BLQ | 130.7 | 83.3 |
| | 24 h post first administration | BLQ | BLQ | BLQ | 45.62 |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| TNF-α (pg/mL) | | | | | |
|---|---|---|---|---|---|
| | Pre-first administration | BLQ | BLQ | BLQ | BLQ |
| | 6 h post first administration | BLQ | BLQ | BLQ | BLQ |
| | 24 h post first administration | BLQ | BLQ | BLQ | BLQ |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| IFN-γ (pg/mL) | | | | | |
|---|---|---|---|---|---|
| | Pre-first administration | BLQ | BLQ | BLQ | BLQ |
| | 6 h post first administration | BLQ | BLQ | BLQ | BLQ |
| | 24 h post first administration | BLQ | BLQ | BLQ | BLQ |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| G-CSF (pg/mL) | | | | | |
|---|---|---|---|---|---|
| | Pre-first administration | BLQ | BLQ | BLQ | BLQ |
| | 6 h post first administration | BLQ | BLQ | BLQ | BLQ |
| | 24 h post first administration | BLQ | BLQ | BLQ | BLQ |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| IL-12p70 (pg/mL) | | | | | |
|---|---|---|---|---|---|
| | Pre-first administration | BLQ | BLQ | BLQ | BLQ |
| | 6 h post first administration | BLQ | BLQ | BLQ | BLQ |
| | 24 h post first administration | BLQ | BLQ | BLQ | BLQ |
| | 72 h post first administration | BLQ | BLQ | BLQ | BLQ |
| | 168 h post first administration | BLQ | BLQ | BLQ | BLQ |
| | 24 h post second administration | BLQ | BLQ | BLQ | BLQ |
| | Pre-third administration | BLQ | BLQ | BLQ | BLQ |
| | 24 h post third administration | BLQ | BLQ | BLQ | BLQ |
| | Pre-fourth administration | BLQ | BLQ | BLQ | BLQ |
| | 24 h post fourth administration | BLQ | BLQ | BLQ | BLQ |
| | 168 h post fourth administration | BLQ | BLQ | BLQ | BLQ |
| | 24 h post fifth administration | BLQ | BLQ | BLQ | BLQ |

| | | | | | |
|---|---|---|---|---|---|
| Note: BLQ in the table stands for below the limit of quantitation. | | | | | |

All of the references mentioned in this invention are incorporated herein by reference as if each individual reference were specifically and individually incorporated by reference. It should also be understood that after reading the foregoing disclosure of the invention, those skilled in the art can make various changes or modifications to the invention, and these equivalent forms are also within the scope of the claims appended to this application.

## Claims

1. A multispecific antibody, which comprises:
a first targeting domain, wherein the first targeting domain comprises one or more LRRC15 antigen-binding domains;
a second targeting domain, wherein the second targeting domain comprises one or more CD40 antigen-binding domains or one or more CD3 antigen-binding domains;
optionally, a third targeting domain that binds to a target protein selected from the group consisting of PD-1, Claudin18.2 (CLDN18.2) and FAP; and/or a fourth targeting domain that binds to TGF-β.

2. The multispecific antibody according to claim 1, wherein the multispecific antibody is a bispecific antibody, a trispecific antibody or a tetraspecific antibody.

3. The multispecific antibody according to claim 2, wherein the bispecific antibody comprises:
a first targeting domain comprising one or more LRRC15 antigen-binding domains; and a second targeting domain comprising one or more CD40 antigen-binding domains or one or more CD3 antigen-binding domains.

4. The multispecific antibody according to claim 2, wherein the trispecific antibody comprises:
a first targeting domain comprising one or more LRRC15 antigen-binding domains;
a second targeting domain comprising one or more CD40 antigen-binding domains; and
a third targeting domain which is a Claudin18.2 antigen-binding domain or a PD-1 antigen-binding domain.

5. The multispecific antibody according to claim 2, wherein the trispecific antibody comprises:
a first targeting domain comprising one or more LRRC15 antigen-binding domains;
a second targeting domain comprising one or more CD3 antigen-binding domains; and
a third targeting domain which is an FAP antigen-binding domain.

6. The multispecific antibody according to claim 2, wherein the tetraspecific antibody comprises:
a first targeting domain comprising one or more LRRC15 antigen-binding domains;
a second targeting domain comprising one or more CD3 antigen-binding domains;
a third targeting domain which is an FAP antigen-binding domain; and
a fourth targeting domain which is a TGF-β antigen-binding domain.

7. The multispecific antibody according to claim 1, wherein the LRRC15 antigen-binding domain comprises three heavy chain variable region CDRs (HCDRs) and three light chain variable region CDRs (LCDRs) selected from the group consisting of:
| Antibody Name | SEQ ID NO of HCDR1 | SEQ ID NO of HCDR2 | SEQ ID NO of HCDR3 | SEQ ID NO of LCDR1 | SEQ ID NO of LCDR2 | SEQ ID NO of LCDR3 |
|---|---|---|---|---|---|---|
| L15-06/ L15-06C | 33 | 34 | 35 | 45 | 46 | 47 |
| L15-08 | 36 | 37 | 38 | 45 | 48 | 49 |
| L15-03 | 39 | 40 | 41 | 50 | 51 | 52 |
| L15-07 . | 42 | 43 | 44 | 50 | 51 | 53 |

8. The multispecific antibody according to claim 1, wherein the CD40 antigen-binding domain comprises a heavy chain variable region having an amino acid sequence set forth in any one of SEQ ID NOs: 11, 13-23 and 114-116, and a light chain variable region having an amino acid sequence set forth in SEQ ID NO: 12; or
a heavy chain variable region having an amino acid sequence set forth in SEQ ID NO: 11, and a light chain variable region having an amino acid sequence set forth in any one of SEQ ID NOs: 12 and 24-26; or
a heavy chain variable region set forth in SEQ ID NO: 27 and a light chain variable region set forth in SEQ ID NO: 28.

9. The multispecific antibody according to claim 1, wherein the CD3 antigen-binding domain comprises a heavy chain variable region having at least 80% sequence identity with the amino acid sequence set forth in any one of SEQ ID NOs: 82-84 and 117, and a light chain variable region having at least 80% sequence identity with the amino acid sequence set forth in SEQ ID NO: 85.

10. The multispecific antibody according to claim 1, wherein the PD-1 antigen-binding domain comprises a heavy chain variable region having at least 80% sequence identity with the amino acid sequence set forth in SEQ ID NO: 29, and a light chain variable region having at least 80% sequence identity with the amino acid sequence set forth in SEQ ID NO: 30.

11. The multispecific antibody according to claim 1, wherein the Claudin18.2 antigen-binding domain comprises a heavy chain variable region having at least 80% sequence identity with the amino acid sequence set forth in SEQ ID NO: 31, and a light chain variable region having at least 80% sequence identity with the amino acid sequence set forth in SEQ ID NO: 32.

12. The multispecific antibody according to claim 1, wherein the FAP antigen-binding domain comprises a heavy chain variable region having at least 80% sequence identity with the amino acid sequence set forth in SEQ ID NO: 86 and a light chain variable region having at least 80% sequence identity with the amino acid sequence set forth in SEQ ID NO: 87; or a heavy chain variable region having at least 80% sequence identity with the amino acid sequence set forth in SEQ ID NO: 88 and a light chain variable region having at least 80% sequence identity with the amino acid sequence set forth in SEQ ID NO: 89; or a heavy chain variable region having at least 80% sequence identity with the amino acid sequence set forth in SEQ ID NO: 90 and a light chain variable region having at least 80% sequence identity with the amino acid sequence set forth in SEQ ID NO: 91; or a heavy chain variable region having at least 80% sequence identity with the amino acid sequence set forth in SEQ ID NO: 92 and a light chain variable region having at least 80% sequence identity with the amino acid sequence set forth in SEQ ID NO: 93.

13. A polynucleotide encoding the multispecific antibody according to any one of claims 1 to 12.

14. Use of the multispecific antibody according to claim 1 in preparation of a medicament for treating cancers/tumors, and/or fibrotic diseases.

15. A pharmaceutical composition, which comprises: (a) the multispecific antibody according to claim 1; and (b) a pharmaceutically acceptable carrier.
